# EUROPEAN PATENT SPECIFICATION

(11) **EP 1 863 801 B1**
(45) Date of publication and mention of the grant of the patent: **29.09.2010**
(21) Application number: 06731118.3
(22) Date of filing: 29.03.2006
(51) Int. Cl.: C07D 413/10, A61K 31/4245, A61P 3/10

(54) **BENZIMIDAZOLE DERIVATIVE AND USE THEREOF**
BENZIMIDAZOLDERIVAT UND ANWENDUNG DAVON
DERIVE DE BENZIMIDAZOLE ET UTILISATION DE CELUI-CI

(30) Priority: 30.03.2005 JP 2005099788; 06.07.2005 JP 2005198014
(43) Date of publication of application: 12.12.2007
(73) Proprietor: Takeda Pharmaceutical Company Limited, Osaka-shi, Osaka 541-0045 (JP)
(72) Inventor: KUROITA, Takanobu c/o TAKEDA PHARMACEUTICAL COMPANY LIMITED, Osaka-shi, Osaka 532-8686 (JP); OJIMA, Mami, c/oTAKEDA PHARMACEUTICAL COMPANY LIMITED, Osaka-shi, Osaka 532-8686 (JP); BAN, Junko, c/o TAKEDA PHARMACEUTICAL COMPANY LIMITED, Osaka-shi, Osaka 532-8686 (JP)
(74) Representative: Casalonga, Axel
(86) International application number: PCT/JP2006/307170
(87) International publication number: WO 2006/107062

(56) References cited:
- EP-A- 0 520 423
- EP-A- 1 452 176

## Description

### Technical Field

The present invention relates to a novel benzimidazole derivative having superior properties as a pharmaceutical agent, production method thereof and use thereof. More particularly, the present invention relates to a prodrug of a benzimidazole derivative having a particular structure, which exhibits superior pharmacological actions (e.g., a strong and sustained hypotensive action, insulin sensitizing activity and the like) and superior properties (e.g., crystallinity, stability and the like), and which is useful as an agent for the prophylaxis or treatment of circulatory diseases such as hypertension, cardiac diseases (cardiac hypertrophy, cardiac failure, cardiac infarction and the like), nephritis, stroke and the like and metabolic diseases such as diabetes and the like, a production method thereof, use thereof and the like.

### Background Art

Angiotensin II causes vasoconstriction via an angiotensin II receptor on the cell membrane and elevates blood pressure. Therefore, an angiotensin II receptor antagonist can be an effective therapeutic drug for circulatory diseases such as hypertension and the like.

As a preferable chemical structure to express strong angiotensin II antagonistic activity, a structure having an acidic group such as a tetrazolyl group, a carboxyl group and the like on a biphenyl side chain is known, and, as a pharmaceutical compound having such structural characteristics, losartan, eprosartan, candesartan cilexetil, olmesartan medoxomil and the like have been clinically used (Ruth R. Wexler et al., Journal of Medicinal Chemistry, vol. 39, p. 625 (1996), JP-A-4-364171, JP-A-5-78328 and the like). JP-A-5-271228 describes that 2-cyclopropyl-1-{[2'-(5-oxo-4,5-dihydro-1,2,4-oxadiazol-3-yl)biphenyl-4-yl]methyl)-1H-benzimidazole-7-carboxylic acid (compound A) and a methyl ester thereof (compound B), which are compounds wherein an acidic group on a biphenyl side chain is 5-oxo-4,5-dihydro-1,2,4-oxadiazol-3-yl group, exhibit a strong angiotensin II antagonistic activity and hypotensive action by oral administration. In addition, WO03/047573 describes that, of the benzimidazole derivatives described in JP-A-5-271228, a particular compound (2-ethoxy-1-{[2'-(5-oxo-4,5-dihydro-1,2,4-oxadiazol-3-yl)-1,1'-biphenyl-4-yl]methyl}-1H-benzimidazole-7-carboxylic acid: compound C) has an insulin sensitizing activity in addition to an angiotensin II antagonistic activity.

As one of the means for enhancing practical use as a pharmaceutical agent, conversion of a compound having a certain pharmacological activity to a prodrug is known. For example, as a prodrug of carboxylic acid, alkylcarbonyloxymethyl ester, 1-alkylcarbonyloxyethyl ester, alkyloxycarbonyloxymethyl ester, 1-alkyloxycarbonyloxyethyl ester and (5-methyl-2-oxo-1,3-dioxol-4-yl)methyl ester (i.e., medoxomil ester) have been widely used for a compound that shows insufficient expression of activity by oral administration in the development of pharmaceutical products to the present. In addition, Farnesol ester, which is a liposoluble substance of indomethacin, and ethyl ester of an ACE inhibitor are known to afford sustained activity and the like.

WO2005/08038 describes medoxomil ester of compound C.

From the aspects of easiness of isolation and purification, stability in formulation and the like, compounds are preferably in the form of crystals. However, whether a compound crystallizes is generally unpredictable, and it is not known until the compound is in fact synthesized and isolated. On the other hand, crystallization generally decreases solubility of compounds, which in turn generally degrades oral absorbability. Therefore, it is not possible to predict if a crystal having superior properties (a good balance of stability and solubility) as a pharmaceutical compound can be obtained.

### Disclosure of the Invention

The present invention aims at providing a novel compound superior as a pharmaceutical agent for the prophylaxis or treatment of circulatory diseases such as hypertension and the like and metabolic diseases such as diabetes and the like, and the like.

The present inventors have conducted intensive studies in an attempt to find a new compound having a superior pharmacological action and superior physicochemical properties so as to provide a pharmaceutical agent more useful as an agent for the prophylaxis or treatment of circulatory diseases such as hypertension and the like and metabolic diseases such as diabetes and the like, and the like.

As a result, they have found that a prodrug compound having a particular structure and capable of converting to compound A in living body has extremely superior properties as a pharmaceutical agent in that it has unexpectedly superior properties (e.g., physicochemical properties such as crystallinity, stability and the like), unexpectedly strong and sustained hypotensive action and the like, which resulted in the completion of the present invention.

Accordingly, the present invention relates to
1. (5-Methyl-2-oxo-1,3-dioxol-4-yl)methyl 2-cyclopropyl-1-([2'-(5-oxo-4,5-dihydro-1,2,4-oxadiazol-3-yl)biphenyl-4-yl]methyl)-1H-benzimidazole-7-carboxylate.
2. A salt of (5-methyl-2-oxo-1,3-dioxol-4-yl)methyl 2-cyclopropyl-1-{[2'-(5-oxo-4,5-dihydro-1,2,4-oxadiazol-3-yl)biphenyl-4-yl]methyl}-1H-benzimidazole-7-carboxylate.
3. (5-Methyl-2-oxo-1,3-dioxol-4-yl)methyl 2-cyclopropyl-1-{[2'-(5-oxo-4.5-dihydro-1,2,4-oxadiazol-3-yl)biphenyl-4-yl]methyl}-1H-benzimidazole-7-carboxylate potassium salt.
4. A solvate of (5-methyl-2-oxo-1,3-dioxol-4-yl)methyl 2-cyclopropyl-1-{[2'-(5-oxo-4,5-dihydro-1,2,4-oxadiazol-3-yl)biphenyl-4-yl]methyl}-1H-benzimidazole-7-carboxylate.
5. A crystal of the compound of any of claims 1 to 4.
6. A method for producing (5-methyl-2-oxo-1,3-dioxol-4-yl)methyl 2-cyclopropyl-1-{[2'-(5-oxo-4,5-dihydro-1,2,4-oxadiazol-3-yl)biphenyl-4-yl]methyl}-1H-benzimidazole-7-carboxylate or a salt thereof, which comprises reacting a reactive derivative of 2-cyclopropyl-1-{[2'-(5-oxo-4,5-dihydro-1,2,4-oxadiazol-3-yl)biphenyl-4-yl]methyl}-1H-benzimidazole-7-carboxylic acid, or a salt thereof, with 4-hydroxymethyl-5-methyl-1,3-dioxol-2-one or a salt thereof.
7. A pharmaceutical agent comprising the compound of any of claims 1 to 4.
8. The pharmaceutical agent of claim 7, which is an angiotensin II antagonist.
9. The pharmaceutical agent of claim 7, which is an agent for the prophylaxis or treatment of circulatory diseases.
10. An insulin sensitizer comprising the compound of any of claims 1 to 4.
11. An enhancer of a hypoglycemic activity of an insulin sensitizer, which comprises the compound of any of claims 1 to 4.
12. A compound according to claim 1 to 4 for its use to antagonize angiotensin II in a mammal.
13. Use of the compound of any of claims 1 to 4 for the production of an angiotensin II antagonist.
14. A compound according to any of claims 1 to 4 for its use to prevent or treat circulatory diseases in an animal.
15. Use of the compound of any of claims 1 to 4 for the production of an agent for the prophylaxis or treatment of circulatory diseases.
16. A compound of any of claims 1 to 4 for its use to produce an insuline sensitizer.
17. Use of the compound of any of claims 1 to 4 for the production of an insulin sensitizer.
18. A compound of any of claims 1 to 4 for its use to produce an enhancer of a hypoglycemic activity of an insulin sensitizer.
19. Use of the compound of any of claims 1 to 4 for the production of an enhancer of a hypoglycemic activity of an insulin sensitizer.

The compound of the present invention shows a superior prophylactic or therapeutic effect on circulatory diseases such as hypertension and the like and metabolic diseases such as diabetes and the like.

### Brief Description of the Drawings

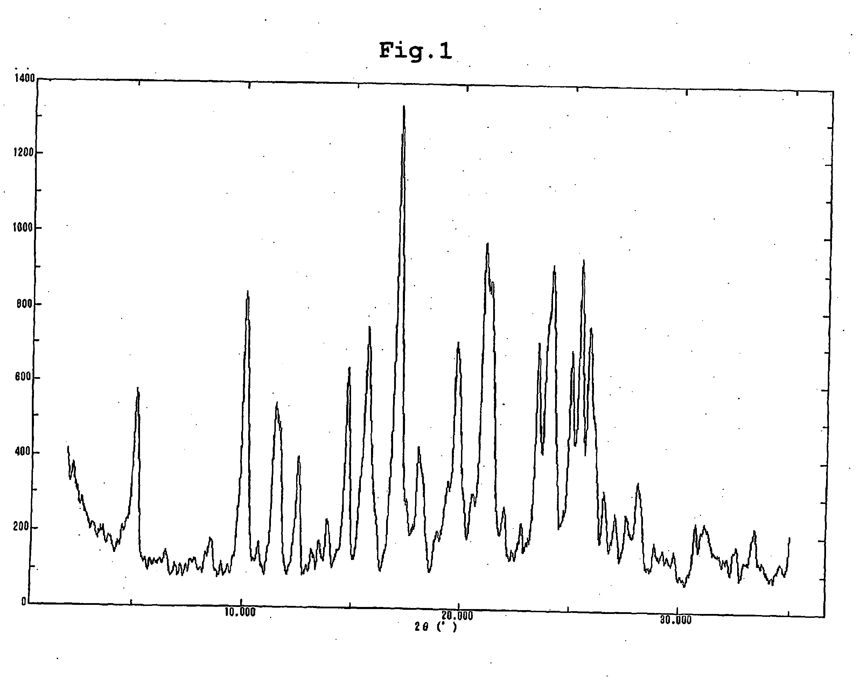

Fig. 1 shows a powder X-ray crystal diffraction pattern of the crystal obtained in Example 3.

### Best Mode for Embodying the Invention

(5-Methyl-2-oxo-1,3-dioxol-4-yl)methyl 2-cyclopropyl-1-{[2'-(5-oxo-4,5-dihydro-1,2,4-oxadiazol-3-yl)biphenyl-4-yl]methyl}-1H-benzimidazole-7-carboxylate (hereinafter sometimes to be referred to as compound (I)) is represented by the formula:

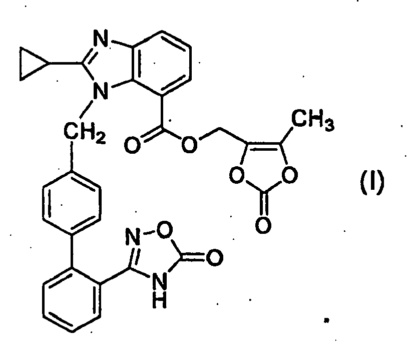

In the formula, a group represented by the formula: 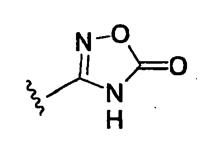 (5-oxo-4,5-dihydro-1,2,4-oxadiazol-3-yl group) includes three tautomers (a', b' and c') represented by the formulas: 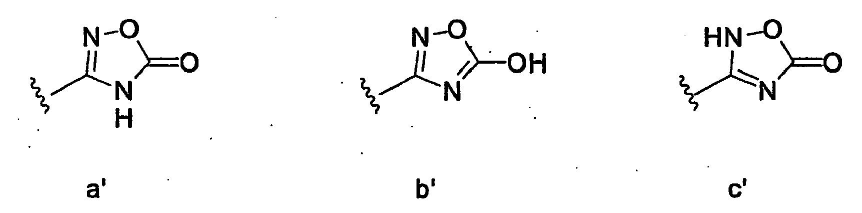 and a 5-oxo-4,5-dihydro-1,2,4-oxadiazol-3-yl group encompasses all of the above-mentioned a', b' and c'.

A salt of compound (I) may be any as long as it is a pharmacologically acceptable salt. As such salt, salts of compound (I) with an inorganic base (e.g., alkali metals such as sodium, potassium and the like; alkaline earth metals such as calcium, magnesium and the like; etc.), an organic base (e.g., organic amines such as tromethamine[tris(hydroxymethyl)methylamine], ethanolamine, trimethylamine, triethylamine, tert-butylamine, pyridine, picoline, diethanolamine, triethanolamine, dicyclohexylamine, N,N'-dibenzylethylenediamine and the like; basic amino acids such as arginine, lysine, ornithine and the like; etc.), ammonia and the like, can be mentioned.

As a salt of compound (I), alkali metal salts of compound (I) are preferable. Of these, a potassium salt is particularly preferable.

Compound (I) may be labeled with an isotope (e.g. ³H, ¹⁴C, ³⁵S, ¹²⁵I and the like) and the like.

Compound (I) may be a crystal, and may have a form of a single crystal or a form of a mixture of plural crystals. The crystals can be produced by crystallization according to a crystallization method known *per se*.

Compound (I) is preferably a crystal, and particularly, Form A crystal having a good balance of stability and solubility and suitable for industrial manufacture is preferable.

Compound (I) may be a solvate (e.g., hydrate etc.) and compound (I) encompasses both solvate and non-solvate (e.g., non-hydrate etc.).

### Production method

Compound (I) can be produced according to, for example, methods shown in the following, a method analogous thereto and the like.

While the yield of compound (I) obtained by the following method may vary depending on the reaction conditions used, compound (I) can be obtained easily at a high purity by conventional means of separation or purification (e.g., recrystallization, column chromatography and the like) from the product by such methods.

Compound (I) can be produced by reacting a reactive derivative (for example, a mixed acid anhydride, an acid halide and the like) of a compound represented by the formula (II) (compound A) or a salt thereof (hereinafter sometimes to be referred to as compound (I-I)) with the corresponding alcohol (IV) (HO-R²) or a salt thereof.

### Method a

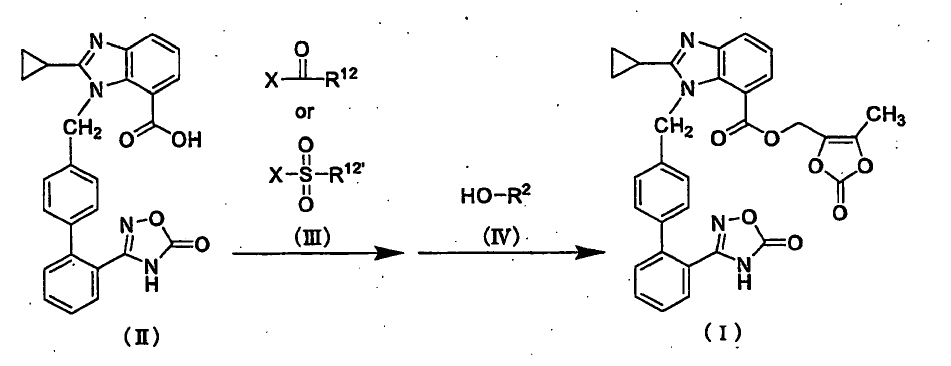

wherein X is a halogen atom (chlorine, bromine, iodine etc.), R² is a (5-methyl-2-oxo-1,3-dioxol-4-yl)methyl group, R¹² is an alkyl group (e.g., a C₁₋₆ alkyl group such as methyl, ethyl, propyl, tert-butyl and the like), an alkoxy group (e.g., a C₁₋₆ alkoxy group such as methoxy, ethoxy, isobutyloxy and the like) or a phenyl group optionally substituted by a halogen atom, a C₁₋₆ alkyl group, a nitro group and the like, and R^{12'} is an alkyl group (e.g., a C₁₋₆ alkyl group such as methyl, ethyl, propyl, tert-butyl and the like), or a phenyl group optionally substituted by a halogen atom, a C₁₋₆ alkyl group, a nitro group and the like.

Method a comprises reacting compound (II) with acylating agent (III) in the presence of a base to give a mixed acid anhydride and reacting the mixed acid anhydride with alcohol (IV) (HO-R²) in the presence of a base to allow esterification.

The mixed acid anhydride is produced in a solvent using about 1 - 3 mol of a base and about 1 - 3 mol of acylating agent (III), relative to 1 mol of compound (II). Subsequently, alcohol (IV) is added to allow reaction, or after once filtering off the salt (salt of the base with H-X), concentrating the filtrate and diluting the residue with a solvent, alcohol (IV) and a base are added to allow reaction, thereby to perform esterification. In the esterification, the amount of alcohol (IV) to be used is about 1 - 3 mol relative to 1 mol of compound (II), and the amount of the base to be used is about 1 - 3 mol relative to 1 mol of compound (II).

As the base, triethylamine, diisopropylethylamine, DBU, 4-dimethylaminopyridine, sodium hydride, potassium tert-butoxide, potassium carbonate, sodium carbonate and the like can be used.

As acylating agent (III), pivaloyl chloride, ethyl chlorocarbonate, isobutyl chlorocarbonate, or acid halides such as 2,4,6-trichlorobenzoyl chloride, 2,4-dichlorobenzoyl chloride, 2,4,6-tribromobenzoyl chloride, 2,3,6-trimethyl-4,5-dinitrobenzoyl chloride and the like; sulfonyl halides such as p-toluenesulfonyl chloride, methanesulfonyl chloride and the like, and the like, which are described in Bulletin of the Chemical Society of Japan, vol. 52, pp. 1989-1993 (1979), are used.

As the solvent, generally, dichloromethane, chloroform, 1,2-dichloroethane, ethyl acetate, tetrahydrofuran, toluene, acetonitrile, acetone, ethyl methyl ketone, 1,4-dioxane, N,N-dimethylformamide, N,N-dimethylacetamide, dimethyl sulfoxide and the like can be used.

While the reaction conditions for producing the mixed acid anhydride vary depending on the combination of the base, acylating agent (III) and the solvent to be used, the reaction is generally preferably carried out at about -30°C to room temperature for about 1 - 10 hrs. While the reaction conditions for the esterification vary depending on the combination of the mixed acid anhydride produced and the solvent to be used, the reaction is generally preferably carried out at about -30°C to the solvent refluxing temperature for about 1 - 10 hrs.

### Method b

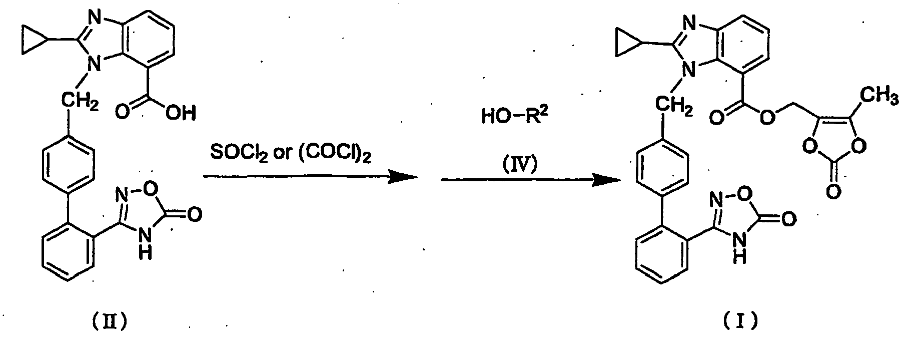

wherein R² is as defined above.

Method b comprises reacting compound (II) with thionyl chloride or oxalyl chloride in the presence of a catalyst such as DMF and the like to give an acid chloride, and reacting the acid chloride with alcohol (IV) (HO-R²) in the presence of a base to allow esterification.

The acid chloride is produced using about 1 - 3 mol of thionyl chloride or oxalyl chloride relative to 1 mol of compound (II) in the presence of a catalytic amount of DMF, in a solvent where necessary. After subsequent concentration, a solvent is added and then alcohol (IV) and the base are added to' allow reaction to perform esterification. In the esterification, the amount of alcohol (IV) to be used is about 1 - 3 mol relative to 1 mol of compound (II), and the amount of the base to be used is about 1 - 3 mol relative to 1 mol of compound (II).

As the base, those similar to the bases used in Method a and the like are used.

As the solvent, those similar to the solvents used in' Method a and the like are used.

While the reaction conditions for producing the acid chloride vary depending on the solvent to be used, the reaction is generally preferably carried out at about -30°C to the solvent refluxing temperature for about 10 min. to 5 hrs. The reaction conditions for the esterification vary depending on the combination of the acid chloride produced and the solvent to be used, the reaction is generally preferably carried out at about -30°C to the solvent refluxing temperature for about 1 to 10 hrs.

### Method c

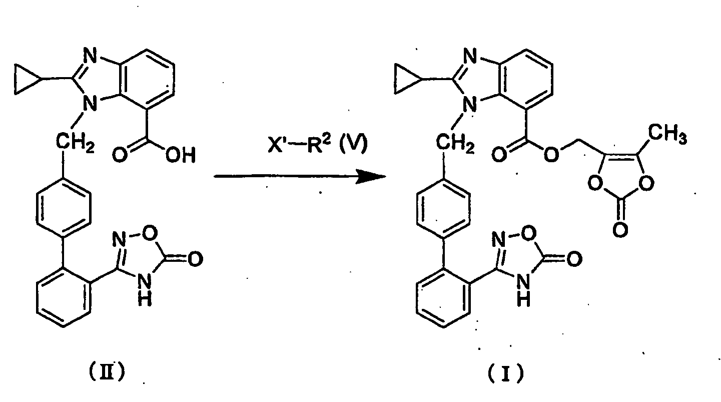

wherein X' is a halogen atom (chlorine, bromine, iodine etc.) and R² is as defined above.

Method c comprises reacting compound (II) (when it is a salt, preferably a salt with an alkali metal such as sodium, potassium and the like; a salt with an alkaline earth metal such as calcium, magnesium and the like; and the like) with alkylating agent (V) (X'-R²) in the presence of a base to allow esterification.

The esterification is carried out in a solvent using about 1 - 3 mol of a base and about 1 - 3 mol of alkylating agent (V), relative to 1 mol of compound (II).

As the base, those similar to the bases used in Method a and the like are used.

As the solvent, those similar to the solvents used in Method a and the like are used.

While the reaction conditions for the esterification vary depending on the combination of the base, alkylating agent (V) and the solvent to be used, the reaction is generally preferably carried out at about -30°C to the solvent refluxing temperature for about 30 min. to 10 hrs.

### Method d

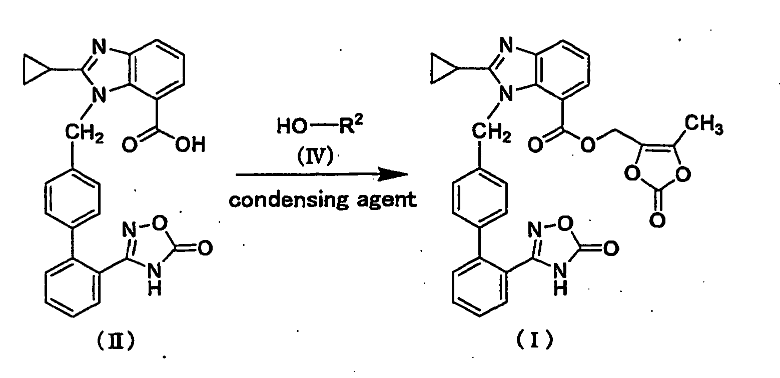

wherein R² is as defined above.

Method d comprises reacting compound (II) with alcohol (IV) (HO-R²) in the presence of a condensing agent to perform esterification.

The esterification is carried out in a solvent using about 1 - 3 mol of the condensing agent and about 1 - 3 mol of alcohol (IV), relative to 1 mol of compound (II).

As the condensing agent, DCC, WSC, Mitsunobu reagents and the like are used.

As the solvent, those similar to the solvents used in Method a and the like are used.

While the reaction conditions for the esterification vary depending on the combination of the condensing agent and solvent to be used, the reaction is generally preferably carried out at about -30°C to the solvent refluxing temperature for about 30 min. to 24 hrs.

Compound (II) can be produced according to method described in JP-A-5-271228 and the like.

When compound (I) is obtained as a free form, it can be converted to an object salt according to a method known per se or a method analogous thereto. Conversely, when it is obtained as a salt, it can be converted to a free form for a different object salt according to a method known per se or a method analogous thereto.

When compound (I) is obtained as an amorphous, it can be crystallized according to a crystallization method known per se or a method analogous thereto.

Form A crystal of compound (I) can be produced by recrystallizing compound (1) from a single solvent of a low-molecular weight aprotic solvent (e.g., acetonitrile, acetone and the like) or a mixed solvent thereof with water to give a solvate crystal of compound (I), and drying the solvate crystal at room temperature - about 150°C, preferably about 80°C - about 120°C, for 5 hr -3 days, preferably 8 hr - 15 hr, under reduce pressure.

Compound (I) and a salt thereof (hereinafter sometimes to be referred to as the compound of the present invention) thus produced show lower toxicity and are safe (in other words, more superior as a pharmaceutical agent from the aspects of acute toxicity, chronic toxicity, genetic toxicity, reproductive toxicity, cardiac toxicity, drug interaction, carcinogenicity and the like), and rapidly converted to compound A in the living body of an animal, particularly a mammal (e.g., human, monkey, cat, pig, horse, bovine, mouse, rat, guinea pig, dog, rabbit etc.).

Compound A or a salt thereof or a prodrug thereof has an insulin sensitizing activity.

The salt of compound A may be any as long as it is a pharmacologically acceptable salt, and those similar to the salts exemplified for compound (I) can be mentioned.

A prodrugof compound A is a compound that converts to compound A due to the reaction by enzyme, gastric acid and the like under the physiological conditions in the body; that is, a compound that converts to compound A by enzymatic oxidation, reduction, hydrolysis and the like, and a compound that converts to compound A by hydrolysis and the like by gastric acid and the like.

Examples of a prodrug of compound A include a compound wherein an amino group of compound A is acylated, alkylated or phosphorylated (e.g., a compound where an amino group of compound A is eicosanoylated, alanylated, pentylaminocarbonylated, (5-methyl-2-oxo-1,3-dioxol-4-yl)methoxycarbonylated, tetrahydrofuranylated, pyrrolidylmethylated, pivaloyloxymethylated or tert-butylated, and the like); a compound wherein a hydroxy group of compound A is acylated, alkylated, phosphorylated or borated (e.g., a compound where a hydroxy group of compound A is acetylated, palmitoylated, propanoylated, pivaloylated, succinylated, fumarylated, alanylated or dimethylaminomethylcarbonylated, and the like); a compound wherein a carboxyl group of compound A is esterified or amidated (e.g., a compound where a carboxyl group of compound A is ethyl esterified, phenyl esterified, carboxymethyl esterified, dimethylaminomethyl esterified, pivaloyloxymethyl esterified, ethoxycarbonyloxyethyl esterified, phthalidyl esterified, (5-methyl-2-oxo-1,3-dioxol-4-yl)methyl esterified, cyclohexyloxycarbonylethyl esterified or methylamidated, and the like) and the like. These compounds can be produced from compound A according to a method known *per se*.

A prodrug of compound A may be a compound that converts to compound A under physiological conditions as described in Development of Pharmaceutical Products, vol. 7, Molecule Design, 163-198, Hirokawa Shoten (1990).

As a prodrug of compound A, a compound where a carboxyl group of compound A is (5-methyl-2-oxo-1,3-dioxol-4-yl)methyl esterified (i.e., compound (I)) is preferable.

When a prodrug of compound A contains an optical isomer, a stereoisomer, a positional isomer or a rotational isomer, these are also encompassed in a prodrug of compound A. For example, when a prodrug of compound A has an optical isomer, an optical isomer resolved from a racemic compound is also encompassed in a prodrug of compound A. These isomers can be obtained as a single product according to a synthetic method and separation method known per se (e.g., concentration, extraction, column chromatography, recrystallization and the like).

A prodrug of compound A may be a crystal, and may have a form of a single crystal or a form of a mixture of plural crystals. The crystals can be produced by crystallization according to a method known *per se*.

Since compound A normalizes the intracellular insulin signal transduction mechanism, which mainly causes insulin resistance, thereby reducing insulin resistance and enhancing insulin action, and has a glucose tolerance improvement action. Therefore, compound A, a salt thereof, or a prodrug thereof containing the compound of the present invention can be used for mammals (e.g., human, monkey, cat, pig, horse, bovine, mouse, rat, guinea pig, dog, rabbit etc.) as an improving agent or an agent for the prophylaxis and/or treatment of the diseases in which insulin resistance is involved. As such diseases, for example, insulin resistance, impaired glucose tolerance; diabetes such as noninsulin dependent diabetes, type II diabetes, type II diabetes associated with insulin resistance, type II diabetes associated with impaired glucose tolerance etc.; various complications such as hyperinsulinemia, hypertension associated with insulin resistance, hypertension associated with impaired glucose tolerance, hypertension associated with diabetes (e.g., type II diabetes etc.), hypertension association with hyperinsulinemia, insulin resistance occurring in association with hypertension, impaired glucose tolerance occurring in association with hypertension, diabetes occurring in association with hypertension, hyperinsulinemia occurring in association with hypertension, diabetic complications [e.g., microangiopathy, diabetic neuropathy, diabetic nephropathy, diabetic retinopathy, diabetic cataract, large vessel disease, osteopenia, diabetic hyperosmolar coma, infectious diseases (e.g., respiratory infectious disease, urinary tract infectious disease, digestive infectious disease, infectious disease of dermal soft tissue, infectious disease of inferior limb etc.), diabetic gangrene, dry mouth, lowered sense of hearing, diabetic cerebrovascular disorder, diabetic peripheric hematogenous disorder, diabetic hypertension and the like], diabetic cachexia and the like; and the like can be mentioned. Compound A, a salt thereof or a prodrug thereof can also be used for treating patients with high normal blood pressure who developed diabetes.

Since compound A has a strong angiotensin II antagonistic activity, the compound of the present invention is useful as an agent for the prophylaxis or treatment of diseases (or diseases whose onset is promoted) developed by the contraction or growth of blood vessels or organ disorder, which expresses via an angiotensin II receptor, or due to the presence of angiotensin II, or a factor induced by the presence of angiotensin II, in mammals (e.g., human, monkey, cat, pig, horse, bovine, mouse, rat, guinea pig, dog, rabbit etc.).

As such diseases, for example, hypertension, blood pressure circadian rhythm abnormality, heart diseases (e.g., cardiac hypertrophy, acute heart failure, chronic heart failure including congestive heart failure, impaired vasodilation, cardiac myopathy, angina pectoris, myocarditis, atrial fibrillation, arrhythmia, tachycardia, cardiac infarction etc.), cerebrovascular disorders (e.g., asymptomatic cerebrovascular disorder, transient cerebral ischemia, apoplexy, cerebrovascular dementia, hypertensive encephalopathy, cerebral infarction etc.), cerebral edema, cerebral circulatory disorder, recurrence and sequela of cerebrovascular disorders (e.g., neurotic symptom, psychic symptom, subjective symptom, disorder in daily living activities etc.), ischemic peripheral circulation disorder, myocardial ischemia, venous insufficiency, progression of cardiac insufficiency after cardiac infarction, renal diseases (e.g., nephritis, glomerulonephritis, glomerulosclerosis, renal failure, thrombotic vasculopathy, complication of dialysis, organ dysfunction including nephropathy by radiation damage etc.), arteriosclerosis including atherosclerosis (e.g., aneurysm, coronary arteriosclerosis, cerebral arteriosclerosis, peripheral arteriosclerosis etc.), vascular hypertrophy, vascular hypertrophy or obliteration and organ disorders after intervention (e.g., percutaneous transluminal coronary angioplasty, stenting, coronary angioscopy, intravascular ultrasound, dounce thrombolytic therapy etc.), vascular re-obliteration and restenosis after bypass, polycythemia, hypertension, organ disorder and vascular hypertrophy after transplantation, rejection after transplantation, ocular diseases (e.g., glaucoma, ocular hypertension etc.), thrombosis, multiple organ disorder, endothelial dysfunction, hypertensive tinnitus, other cardiovascular diseases (e.g., deep vein thrombosis, obstructive peripheral circulatory disorder, arteriosclerosis obliterans, obstructive thromboangiitis, ischemic cerebral circulatory disorder, Raynaud's disease, Berger disease etc.), metabolic and/or nutritional disorders (e.g., obesity, hyperlipidemia, hypercholesterolemia, hyperuricacidemia, hyperkalemia, hypernatremia etc.), nerve degeneration diseases (e.g., Alzheimer's disease, Parkinson's syndrome, amyotrophic lateral sclerosis, AIDS encephalopathy etc.), central nervous system disorders (e.g., cerebral hemorrhage, cerebral infarction, their sequela and complication, head injury, spinal injury, cerebral edema, sensory malfunction, sensory functional disorder, autonomic nervous system disorder, autonomic nervous system malfunction, multiple sclerosis etc.), dementia, defects of memory, disorder of consciousness, amnesia, anxiety symptom, catatonic symptom, discomfort mental state, psychopathies (e.g., depression, epilepsy, alcoholism etc.), inflammatory diseases (e.g., arthritis such as rheumatoid arthritis, osteoarthritis, rheumatoid myelitis, periostitis etc.; inflammation after operation and injury; remission of swelling; pharyngitis; cystitis; pneumonia; atopic dermatitis; inflammatory intestinal diseases such as Crohn's disease, ulcerative colitis etc.; meningitis; inflammatory ocular disease; inflammatory pulmonary disease such as pneumonia, pulmonary silicosis, pulmonary sarcoidosis, pulmonary tuberculosis etc.), allergic diseases (e.g., allergic rhinitis, conjunctivitis, gastrointestinal allergy, pollinosis, anaphylaxis etc.), chronic obstructive pulmonary disease, interstitial pneumonia, pneumocystis carinnii pneumonia, collagen diseases (e.g., systemic lupus erythematodes, scleroderma, polyarteritis etc.), hepatic diseases (e.g., hepatitis including chronic hepatitis, hepatic cirrhosis etc.), portal hypertension, digestive system disorders (e.g., gastritis, gastric ulcer, gastric cancer, gastric disorder after operation, dyspepsia, esophageal ulcer, pancreatitis, colon polyp, cholelithiasis, hemorrhoidal disease, varices ruptures of esophagus and stomach etc.), blood and/or myelopoietic diseases (e.g., erythrocytosis, vascular purpura, autoimmune hemolytic anemia, disseminated intravascular coagulation syndrome, multiple myelopathy etc.), bone diseases (e.g., fracture, refracture, osteoporosis, osteomalacia, bone Paget's disease, sclerosing myelitis, rheumatoid arthritis, osteoarthritis of the knee and joint tissue dysfunction and the like caused by diseases similar to these etc.), solid tumor, tumors (e.g., malignant melanoma, malignant lymphoma, cancer of digestive organs (e.g., stomach, intestine etc.) etc.), cancer and cachexia following cancer, metastasis cancer, endocrinopathy (e.g., Addison's disease, Cushing's syndrome, pheochromocytoma, primary aldosteronism etc.), Creutzfeldt-Jakob disease, urinary organ and/or male genital diseases (e.g., cystitis, prostatic hypertrophy, prostatic cancer, sex infectious disease etc.), female disorders (e.g., climacteric disorder, gestosis, endometriosis, hysteromyoma, ovarian disease, breast disease, sex infectious disease etc.), disease relating to environment and occupational factors (e.g., radiation hazard, hazard by ultraviolet, infrared or laser beam, altitude sickness etc.), respiratory diseases (e.g., cold syndrome, pneumonia, asthma, pulmonary hypertension, pulmonary thrombosis and pulmonary embolism etc.), infectious diseases (e.g., viral infectious diseases with cytomegalovirus, influenza virus, herpes virus etc., rickettsiosis, bacterial infectious disease etc.), toxemias (e.g., sepsis, septic shock, endotoxin shock, Gram-negative sepsis, toxic shock syndrome etc.), otorhinolaryngological diseases (e.g., Meniere's syndrome, tinnitus, dysgeusia, vertigo, disequilibrium, dysphagia etc.), skin diseases (e.g., keloid, hemangioma, psoriasis etc.), intradialytic hypotension, myasthenia gravis, systemic diseases such as chronic fatigue syndrome and the like can be mentioned.

Since the compound of the present invention can maintain a constant hypotensive action both day and night, reduction of the dose and frequency is possible as compared to the administration of compound A. In addition, it can effectively suppress particularly problematic increase in the blood pressure before and after rising in patients with hypertension.

In addition, by longer term sustained suppression of the action of angiotensin II, the compound of the present invention improves disorder or abnormality or suppresses promotion thereof in the biofunction and physiological action, that causes adult disorders and various diseases linked with aging and the like, which in turn leads to the primary and secondary prophylaxis of diseases or clinical conditions caused thereby or suppression of the progression thereof. As the disorder or abnormality in the biofunction and physiological action, for example, disorder or abnormality in automatic controlling capability of cerebral circulation and/or renal circulation, disorder of circulation (e.g., peripheral, cerebral, microcirculation etc.), disorder of blood-brain-barrier, salt susceptibility, abnormal state of coagulation and fibrinolysis system, abnormal state of blood and blood cell components (e.g., accentuation of platelet aggregation activity, erythrocyte deformability, accentuation of leukocyte adhesiveness, rise of blood viscosity etc.), production and function accentuation of growth factor and cytokines (e.g., PDGF, VEGF, FGF, interleukin, TNF-α, MCP-1 etc.), accentuation of proliferation and infiltration of inflammatory cells, accentuation of production of free radical, liposteatosis accentuation, endothelial function disorder, dysfunction of endothelium, cell and organ, edema, cell morphogenesis change of smooth muscle etc. (morphogenesis to proliferation type etc.), production and function accentuation of vasoactive substance and thrombosis inducers (e.g., endothelin, thromboxane A₂ etc.), abnormal constriction of blood vessel etc., metabolic disorder (e.g., serum lipid abnormalities, dysglycemia etc.), abnormal growth of cell etc., angiogenesis (including abnormal vasculogenesis during abnormal capillary reticular formation in adventitial coat of arteriosclerosis) and the like can be mentioned. Of these, the present invention can be used as an agent for the primary and secondary prophylaxis or treatment of organ disorders associated with various diseases (e.g., cerebrovascular disorder and organ disorder associated therewith, organ disorder associated with cardiovascular disease, organ disorder associated with diabetes, organ disorder after intervention etc.). In particular, since compound A has an activity of inhibiting proteinuria, the compound of the present invention can be used as an agent for protecting kidney. Therefore, the compound of the present invention can be advantageously used when the patients with insulin resistance, impaired glucose tolerance, diabetes or hyperinsulinemia have concurrently developed the above-mentioned diseases or clinical condition.

Since compound A has an activity of inhibiting body weight gain, the compound of the present invention can be used as a body weight gain inhibitor to mammals. Target mammals may be any mammals of which body weight gain is to be avoided. The mammals may have a risk of body weight gain genetically or may be suffering from lifestyle-related diseases such as diabetes, hypertension and/or hyperlipidemia etc. The body weight gain may be caused by excessive feeding or diet without nutrient balance, for may be derived from combination drug, for example, insulin sensitizers having PPARγ-agonistic activity such as troglitazone, rosiglitazone, englitazone, ciglitazone, pioglitazone etc. and the like. In addition, body weight gain may be preliminary to obesity, or may be body weight gain of obesity patients. Here, obesity is defined that BMI (body mass index; body weight (kg) / [height (m)]²) is at least twenty-five for Javanese (criterion by Japan Society for the Study of Obesity), or at least thirty for westerner (criterion by WHO).

The Japan Diabetes Society issued a report on the new diabetic criteria in 1999.

According to this report, diabetes is'a condition wherein the fasting blood glucose level (glucose concentration of venous plasma) is not less than 126 mg/dl, the 2-hour value (glucose concentration of venous plasma) of the 75 g oral glucose tolerance test (75 g OGTT) is not less than 200 mg/dl, or the casual blood glucose level (glucose concentration of venous plasma) is not less than 200 mg/dl. In addition, a condition which does not fall under the above-mentioned diabetes, and which is not a "condition where the fasting blood glucose level (glucose concentration of venous plasma) is less than 110 mg/dl or the 2-hour value (glucose concentration of venous plasma) of the 75 g oral glucose tolerance test (75 g OGTT) is less than 140 mg/dl" (normal type), is called a "borderline type".

In addition, regarding diagnostic criteria for diabetes, new diagnostic criteria were reported by ADA (The American Diabetes Association) in 1997 and by WHO in 1998.

According to these reports, diabetes is a condition where the fasting blood glucose level (glucose concentration in venous plasma) is not less than 126 mg/dl, and the 2-hour value (glucose concentration in venous plasma) of the 75 g oral glucose tolerance test is not less than 200 mg/dl.

In addition, according to the above reports, impaired glucose tolerance is a condition where the fasting blood glucose level (glucose concentration in venous plasma) is less than 126 mg/dl, and the 2-hour value (glucose concentration in venous plasma) of the 75 g oral glucose tolerance test is not less than 140 mg/dl and less than 200 mg/dl. Furthermore, according to the ADA report, a condition where the fasting blood glucose level (glucose concentration in venous plasma) is not less than 110 mg/dl and less than 126 mg/dl, is called IFG (Impaired Fasting Glucose). On the other hand, according to the WHO report, of the conditions of IFG (Impaired Fasting Glucose), a condition where the 2-hour value (glucose concentration in venous plasma) of the 75 g oral glucose tolerance test is less than 140 mg/dl, is called IFG (Impaired Fasting Glycemia).

The compound of the present invention can be used as an improving agent or an agent for the prophylaxis or treatment of diabetes, borderline type, impaired glucose tolerance, IFG (Impaired Fasting Glucose) and IFG (Impaired Fasting Glycemia) as defined by the above-mentioned new diagnostic criteria. Furthermore, the compound of the present invention can be also used as a therapeutic agent for hypertension of hypertensive patients showing a level not less than the above-mentioned diagnostic criteria (e.g., fasting blood glucose level of 126 mg/dl). Moreover, the compound of the present invention can be also used to prevent the progression of the borderline type, impaired glucose tolerance, IFG (Impaired Fasting Glucose) or IFG (Impaired Fasting Glycemia) to diabetes.

The compound of the present invention is useful as an agent for suppression or improvement of cardiac depression, progression of cardiac remodeling and exacerbation of symptoms, or an agent for suppression of decrease in survival rate, of patients with heart disease (e.g., cardiac hypertrophy, acute heart failure, chronic heart failure including congestive heart failure, impaired vasodilation, cardiac myopathy, angina pectoris, myocarditis, atrial fibrillation, arrhythmia, tachycardia, cardiac infarction etc.) in association with diabetes. The compound of the present invention is effective for the prevention of the onset of heart diseases (e.g., cardiac hypertrophy, acute heart failure, chronic heart failure including congestive heart failure, impaired vasodilation, cardiac myopathy, angina pectoris, myocarditis, atrial fibrillation, arrhythmia, tachycardia, cardiac infarction etc.) and cerebrovascular disorders (e.g., asymptomatic cerebrovascular disorder, transient cerebral ischemia, apoplexy, cerebrovascular dementia, hypertensive encephalopathy, cerebral infarction etc.) of diabetic patients.

The compound of the present invention is useful as an agent for the prophylaxis or treatment of metabolic syndrome. Because patients with metabolic syndrome have an extreme high incidence of cardiovascular diseases as compared to patients with single lifestyle-related diseases, the prophylaxis or treatment of metabolic syndrome is quite important to prevent cardiovascular diseases.

The criteria for diagnosis of metabolic syndrome are announced by WHO in 1999, and by NCEP in 2001. According to the criterion of WHO, patients with at least two of abdominal obesity, dyslipidemia (high serum triglycerides or low HDL cholesterol), hypertension in addition to hyperinsulinemia or fasting blood glucose are diagnosed as metabolic syndrome (World Health Organization: Definition, Diagnosis and Classification of Diabetes Mellitus and Its Complications. Part I: Diagnosis and Classification of Diabetes Mellitus, World Health Organization, Geneva, 1999). According to the criterion of Adult Treatment. Panel III of National Cholesterol Education Program, that is an indicator for managing ischemic heart diseases in America, patients with at least three of abdominal obesity, high triglycerides, low HDL cholesterol, hypertension and fasting blood glucose are diagnosed as metabolic syndrome (National Cholesterol Education Program: Executive Summary of the Third Report of National Cholesterol Education Program (NCEP) Expert Panel on Detection, Evaluation, and Treatment of High Blood Cholesterol in Adults (Adults Treatment Panel III). The Journal of the American Medical Association, Vol. 285, 2486-2497, 2001).

The compound of the present invention can be used for treating patients of high blood pressure with metabolic syndrome.

Since compound A has an anti-inflammatory action, the compound of the present invention can be used as an anti-inflammatory agent for preventing or treating inflammatory diseases. Examples of inflammatory diseases include inflammatory diseases due to various diseases such as arthritis (e.g. rheumatoid arthritis, osteoarthritis, rheumatoid myelitis, gouty arthritis, synovitis), asthma, allergic diseases, arteriosclerosis including atherosclerosis (aneurysm, coronary sclerosis, cerebral arterial sclerosis, peripheral arterial sclerosis etc.), digestive tract disease such as inflammatory intestine disease (e.g. Crohn's disease, ulcerative colitis), diabetic complication (diabetic nerves disorder, diabetic vascular disorder), atopic dermatitis, chronic obstructive pulmonary disease, systemic lupus erythematosus, visceral inflammatory disease (nephritic, hepatitis), autoimmune hemolytic anemia, psoriasis, nervous degenerative disease (e.g. Alzheimer's disease, Parkinson's diseases, amyotrophic lateral sclerosis, AIDS encephalopathy), central nervous disorder (e.g. cerebrovascular disorder such as cerebral hemorrhage and cerebral infarct, head trauma, spinal damage, cerebral edema, multiple sclerosis), meningitis, angina, cardiac infarct; congestive cardiac failure, vascular hypertrophy or occlusion and organ disorder after intervention (transdermal coronary plasty, stent indwelling, coronary endoscope, intravascular ultrasound, intracoronary thrombolysis etc), vascular reocculusion or restenosis after bypass operation, endothelial functional disorder, other circulatory disease (intermittent claudication, obstructive peripheral circulatory disorder, obstructive arteriosclerosis, obstructive thrombotic angitis, ischemic cerebral circulatory disorder, Leiner's disease, Buerger's disease), inflammatory ocular disease, inflammatory pulmonary disease (e.g. chronic pneumonia, silicosis, pulmonary sarcoidosis, pulmonary tuberculosis), endometritis, toxemia (e.g. sepsis, septic shock, endotoxin shock, gram negative sepsis, toxin shock syndrome), cachexia (e.g. cachexia due to infection, carcinomatous cachexia, cachexia due to acquired immunodeficiency syndrome), cancer, Addison's disease, Creutzfeldt-Jakob disease, virus infection (e.g. infection of virus such as cytomegalovirus, influenza virus, herpes etc.), disseminated intravascular coagulation.

In addition, since compound A has an analgesic action, the compound of the present invention can be also used as an analgesic agent for preventing or treating pain. Examples of pain diseases include acute pain due to inflammation, plain associated with chronic inflammation, pain associated with acute inflammation, pain after operation (pain of incisional, deep pain, organ pain, chronic pain after operation etc.), muscular pain (muscular pain associated with chronic pain disease, shoulder stiffness etc.), arthralgia, toothache, gnathicarthralgia, headache (migraine, catatonic headache, headache associated with fever, headache associated hypertension), organ pain (cardiac pain, angina pain, abdominal pain, renal pain, ureterane pain, bladder pain), pain in obstetrics area (mittelschmerz, dysmenorrheal, labor pain), neuralgia, (disc hernia, nerve root pain, neuralgia after herpes zoster, trigeminal neuralgia), carcinomatous pain, reflex sympathetic atrophy, complex local pain syndrome, and the like. The compound of the present invention is effective in alleviate directly and rapidly various pains such as nervous pain, carcinomatous pain and inflammatory pain, and exhibits the particularly excellent analgesic effect to patients and pathologies in which a pain sense threshold is lowered.

The compound of the present invention is particularly useful as an analgesic agent for pain associated with chronic inflammation or pain associated with hypertension, or as an agent for preventing or treating inflammatory disease or pain due to (1) arteriosclerosis including atherosclerosis, (2) vascular hypertrophy, occlusion or organ disorder after intervention, (3) reocclusion, restenosis or endothelial functional disorder after bypass operation, (4) intermittent claudication, (5) occlusive peripheral circulatory disorder, (6) occlusive arteriosclerosis.

The compound of the present invention can be used as a safe pharmaceutical agent to mammals (e.g., human, monkey, cat, swine, horse, bovine, mouse, rat, guinea pig, dog, rabbit and the like) in the form of the compound as it is or a pharmaceutical composition after admixing with a pharmacologically acceptable carrier according to a method known per se.

As used herein, as the pharmacologically acceptable carrier, various organic or inorganic carrier substances conventionally used as materials for preparations can be used. For example, excipient, lubricant, binder and disintegrant for solid preparations; solvent, dissolution aids, suspending agent, isotonizing agent and buffer for liquid preparations; and the like can be mentioned. Where necessary, additives for preparation, such as preservative, antioxidant, coloring agent, sweetening agent and the like, can be also used.

Preferable examples of excipient include lactose, sucrose, D-mannitol, D-sorbitol, starch, pregelatinized starch, dextrin, crystalline cellulose, low-substituted hydroxypropylcellulose, sodium carboxymethylcellulose, gum arabic, pullulan, light silicic anhydride, synthetic aluminum silicate, magnesium aluminometasilicate and the like.

Preferable examples of lubricant include magnesium stearate, calcium stearate, talc, colloidal silica and the like.

Preferable examples of binder include pregelatinized starch, sucrose, gelatin, gum arabic, methylcellulose, carboxymethylcellulose, sodium carboxymethylcellulose, crystalline cellulose, sucrose, D-mannitol, trehalose, dextrin, pullulan, hydroxypropylcellulose, hydroxypropylmethylcellulose, polyvinylpyrrolidone and the like.

Preferable examples of disintegrant include lactose, sucrose, starch, carboxymethylcellulose, calcium carboxymethylcellulose, sodium croscarmellose, sodium carboxymethyl starch, light silicic anhydride, low-substituted hydroxypropylcellulose and the like.

Preferable examples of solvent include water for injection, physiological brine, Ringer's solution, alcohol, propylene glycol, polyethylene glycol, sesame oil, corn oil, olive oil, cottonseed oil and the like.

Preferable examples of dissolution aids include polyethylene glycol, propylene glycol, D-mannitol, trehalose, benzyl benzoate, ethanol, tris-aminomethane, cholesterol, triethanolamine, sodium carbonate, sodium citrate, sodium salicylate, sodium acetate and the like.

Preferable examples of suspending agent include surfactants such as stearyltriethanolamine, sodium lauryl sulfate, lauryl aminopropionate, lecithin, benzalkonium chloride, benzethonium chloride, glycerol monostearate etc.; hydrophilic polymers such as polyvinyl alcohol, polyvinylpyrrolidone, sodium carboxymethylcellulose, methylcellulose, hydroxymethylcellulose, hydroxyethylcellulose, hydroxypropylcellulose etc.; polysorbates, polyoxyethylene hydrogenated castor oil and the like.

Preferable examples of isotonizing agent include sodium chloride, glycerin, D-mannitol, D-sorbitol, glucose and the like.

Preferable examples of buffer include buffers such as phosphate, acetate, carbonate, citrate etc., and the like.

Preferable examples of preservative include p-oxybenzoate, chlorobutanol, benzyl alcohol, phenethyl alcohol, dehydroacetic acid, sorbic acid and the like.

Preferable examples of antioxidant include sulfite, ascorbate and the like.

Preferable examples of coloring agent include water-soluble edible tar dyes (e.g., food colors such as Food Red Nos. 2 and 3, Food Yellow Nos. 4 and 5, Food Blue Nos. 1 and 2 etc.), water-insoluble Lake dyes (e.g., aluminum salts of the aforementioned water-soluble edible tar dyes etc.), natural colors (e.g., β-carotene, chlorophyll, iron oxide red etc.) and the like.

Preferable examples of sweetening agent include sodium saccharin, dipotassium glycyrrhizinate, aspartame, stevia and the like.

The dosage form of the pharmaceutical composition includes, for example, oral agents such as tablet, capsule (including soft capsule and microcapsule), granule, powder, syrup, emulsion, suspension, sustained-release preparation and the like, which can be each safely administered orally.

The pharmaceutical composition can be prepared by conventional methods in the field of pharmaceutical manufacturing technical field, such as methods described in the Japanese Pharmacopoeia, and the like. Specific production methods for such preparations are hereinafter described in detail.

For example, a tablet is produced by adding, for example, an excipient (e.g., lactose, sucrose, starch, D-mannitol etc.), a disintegrant (e.g., calcium carboxymethylcellulose etc.), a binder (e.g., pregelatinized starch, gum arabic, carboxymethylcellulose, hydroxypropylcellulose, polyvinylpyrrolidone etc.), a lubricant (e.g., talc, magnesium stearate, polyethylene glycol 6.000 etc.) and the like, to the active ingredient, compression-shaping, and, where necessary, applying a coating by a method known *per se* using a coating base known *per se* for the purpose of achieving taste masking, enteric dissolution or sustained release.

The capsule can be made as a hard capsule filled with a powder or granular pharmaceutical agent, or a soft capsule filled with a liquid or suspension liquid. The hard capsule is produced by mixing and/or granulating an active ingredient with, for example, an excipient (e.g., lactose, sucrose, starch, crystalline cellulose, D-mannitol and the like), a disintegrant (low substituted hydroxypropylcellulose, carmellose calcium, corn starch, sodium croscarmellose and the like), a binder (hydroxypropylcellulose, polyvinylpyrrolidone, hydroxypropylmethylcellulose and the like), a lubricant (magnesium stearate and the like) and the like, and filling the mixture or granule in a capsule formed from the aforementioned gelatin, hydroxypropylmethylcellulose and the like. The soft capsule is produced by dissolving or suspending the active ingredient in a base (soybean oil, cottonseed oil, medium chain fatty acid triglyceride, beeswax and the like) and sealing the prepared solution or suspension in a gelatin sheet using, for example, a rotary filling machine and the like.

When the compound of the present invention is a salt and avoidance of contact of the compound of the present invention in the form of a salt with water is preferable, the compound of the present invention is preferably dry-mixed with an excipient and the like to give a hard capsule.

The content of the compound of the present invention in a pharmaceutical composition is generally about 0.01 - about 99.9 wt%, preferably about 0.1 - about 50 wt%, relative to the entire preparation.

The dose of the compound of the present invention is determined in consideration of age, body weight, general health condition, sex, diet, administration time, administration method, clearance rate, combination of drugs, the level of disease for which the patient is under treatment then, and other factors.

While the dose varies depending on the target disease, condition, subject of administration, administration method and the like, for oral administration as a therapeutic agent for essential hypertension in adult, the daily dose of 0.1 - 100 mg is preferably administered in a single dose or in 2 or 3 portions.

In addition, because the compound of the present invention is superior in safety, it can be administered for a long period.

The compound of the present invention can be used in combination with pharmaceutical agents such as a therapeutic agent for diabetes, a therapeutic agent for diabetic complications, an anti-hyperlipidemia agent, an anti-arteriosclerotic agent, an anti-hypertensive agent, an anti-obesity agent, a diuretic, an antigout agent, an antithrombotic agent, an anti-inflammatory agent, a chemotherapeutic agent, an immunotherapeutic agent, a therapeutic agent for osteoporosis, an anti-dementia agent, an erectile dysfunction amelioration agent, a therapeutic agent for urinary incontinence/urinary frequency and the like (hereinafter to be abbreviated as a combination drug). On such occasions, the timing of administration of the compound of the present invention and that of the combination drug is not limited, as long as the compound of the present invention and the combination drug are combined. As the mode of such administration, for example, (1) administration of a single preparation obtained by simultaneous formulation of the compound of the present invention and a combination drug, (2) simultaneous administration of two kinds of preparations obtained by separate formulation of the compound of the present invention and a combination drug, by a single administration route, (3) time staggered administration of two kinds of preparations obtained by separate formulation of the compound of the present invention and a combination drug, by the same administration route, (4) simultaneous administration of two kinds of preparations obtained by separate formulation of the compound of the present invention and a combination drug, by different administration routes, (5) time staggered administration of two kinds of preparations obtained by separate formulation of the compound of the present invention and a combination drug, by different administration routes, such as administration in the order of the compound of the present invention and then the combination drug, or administration in a reversed order, and the like can be mentioned. The dose of the combination drug can be appropriately determined based on the dose clinically employed. The mixing ratio of the compound of the present invention and the combination drug can be appropriately selected according to the administration subject, administration route, target disease, condition, combination, and other factors. In cases where the administration subject is human, for example, the combination drug may be used in an amount of 0.01 to 100 parts by weight per part by weight of the compound of the present invention.

As the therapeutic agent for diabetes, for example, insulin preparations (e.g., animal insulin preparations extracted from the bovine or swine pancreas; human insulin preparations synthesized by a genetic engineering technique using E. coli or a yeast, and the like), other insulin sensitizers (e.g., pioglitazone hydrochloride, troglitazone, rosiglitazone, GI-262570, JTT-501, MCC-555, YM-440, KRP-297, CS-011, FK-614 etc.), α-glucosidase inhibitors (e.g., voglibose, acarbose, miglitol, emiglitate etc.), biguanides (e.g., phenformin, metformin, buformin etc.), insulin secretagogues [e.g., sulfonylureas (e.g., tolbutamide, glibenclamide, gliclazide, chlorpropamide, tolazamide, acetohexamide, glyclopyramide, glimepiride, glipizide, glybuzole etc.), repaglinide, senaglinide, nateglinide, mitiglinide or its calcium salt hydrate, GLP-1 etc.], amyrin agonists (e.g., pramlintide etc.), phosphotyrosine phosphatase inhibitors (e.g., vanadic acid etc.), dipeptidylpeptidase IV inhibitors (e.g., NVP-DPP-278, PT-100, P32/98 etc.), β3 agonists (e.g., CL-316243, SR-58611-A, UL-TG-307, SB-226552, AJ-9677, BMS-196085, AZ40140 etc.), gluconeogenesis inhibitors (e.g., glycogen phosphorylase inhibitor, glucose-6-phosphatase inhibitor, glucagon antagonist etc.), SGLT (sodium-glucose cotransporter) inhibitors (e.g., T-1095 etc.) and the like, can be mentioned.

As the therapeutic agents for diabetic complications, for example, aldose reductase inhibitors (e.g., tolrestat, epalrestat, zenarestat, zopolrestat, minalrestat, fidarestat, SNK-860, CT-112 etc.), neurotrophic factors (e.g., NGF, NT-3, BDNF etc.), PKC inhibitors (e.g., LY-333531 etc.), AGE inhibitors (e.g., ALT946, pimagedine, pyratoxathine, N-phenacylthiazolium bromide (ALT766), EXO-226 etc.), active oxygen scavengers (e.g., thioctic acid etc.), cerebral vasodilators (e.g., tiapride, mexiletine etc.) and the like can be mentioned.

As the anti-hyperlipidemia agents, for example, statin compounds which are cholesterol synthesis inhibitors (e.g., cerivastatin, pravastatin, simvastatin, lovastatin, atorvastatin, fluvastatin, itavastatin or salts thereof (e.g., sodium salt etc.) etc.), squalene synthetase inhibitors (e.g. TAK-475 etc.), fibrate compounds having a triglyceride lowering effect (e.g., bezafibrate, clofibrate, simfibrate, clinofibrate etc.), EPA, DHA and the like can be mentioned.

As the anti-arteriosclerotic agents, for example, an acyl-Coenzyme A cholesterol acyltransferase (ACAT) inhibitor (e.g. melinamide, CS-505 etc.), a lipid rich plaque regressing agent (e.g. compounds described in WO 02/06264, WO 03/059900 etc.) and the like can be mentioned.

As the antihypertensive agents, for example, angiotensin converting enzyme inhibitors (e.g., captopril, enalapril, delapril etc.), angiotensin II antagonists (e.g., candesartan cilexetil, candesartan, losartan, losartan potassium, eprosartan, valsartan, termisartan, irbesartan, tasosartan, olmesartan, olmesartan medoxomil etc.), calcium antagonists (e.g., manidipine, nifedipine, amlodipine, efonidipine, nicardipine etc.), β-blocker (e.g., metoprolol, atenolol, propranolol, carvedilol, pindolol etc.), clonidine and the like can be mentioned.

As the anti-obesity agents, for example, central acting anti-obesity agent (e.g., dexfenfluramine, fenfluramine, phentermine, sibutramine, amfepramone, dexamphetamine, mazindol, phenylpropanolamine, clobenzorex etc.), pancreatic lipase inhibitors (e.g., orlistat etc.), β3 agonist (e.g., CL-316243, SR-58611-A, UL-TG-307, SB-226552, AJ-9677, BMS-196085, AZ40140 etc.), anorectic peptides (e.g., leptin, CNTF (ciliary neurotrophic factor) etc.), cholecystokinin agonists (e.g., lintitript, FPL-15849 etc.) and the like can be mentioned.

As the diuretics, for example, xanthine derivatives (e.g., theobromine and sodium salicylate, theobromine and calcium salicylate etc.), thiazide preparations (e.g., ethiazide, cyclopenthiazide, trichlormethiazide, hydrochlorothiazide, hydroflumethiazide, benzylhydrochlorothiazide, penfluthiazide, polythiazide, methyclothiazide etc.), anti-aldosterone preparations (e.g., spironolactone, triamterene etc.), carbonic anhydrase inhibitors (e.g., acetazolamide etc.), chlorobenzenesulfonamide preparations (e.g., chlortalidone, mefruside, indapamide etc.), azosemide, isosorbide, ethacrynic acid, piretanide, bumetanide, furosemide and the like can be mentioned.

As the antigout agents, for example, allopurinol, probenecid, colchicine, benzbromarone, febuxostat, citrate and the like can be mentioned.

As the antithrombotic agents, for example, anticoagulating agent [e.g., heparin sodium, heparin potassium, warfarin potassium (warfarin), activated blood coagulation factor X inhibitor (e.g., compounds described in WO 2004/048363 etc.)], thrombolytic agent [e.g., tPA, urokinase], antiplatelet agent [e.g., aspirin, sulfinpyrazone (anturan), dipyridamole (persantin), ticlopidine (panaldine), cilostazol (pletal), GPIIb/IIIa antagonist (ReoPro), clopidogrel etc.], and the like can be mentioned.

As the antiinflammatory agents, for example, non-steroidal antiinflammatory agents, such as acetaminophen, fenasetin, ethenzamide, sulpyrine, antipyrine, migrenin, aspirin, mefenamic acid, flufenamic acid, diclofenac sodium, loxoprofen sodium, phenylbutazone, indomethacin, ibuprofen, ketoprofen, naproxen, oxaprozin, flurbiprofen, fenbufen, pranoprofen, floctafenine, epirizol, tiaramide hydrochloride, zaltoprofen, gabexate mesylate, camostat mesylate, ulinastatin, colchicine, probenecid, sulfinpyrazone, benzbromarone, allopurinol, sodium gold thiomalate, sodium hyaluronate, sodium salicylate, morphine hydrochloride, salicylic acid, atropine, scopolamine, morphine, pethidine, levorphanol, ketoprofen, naproxen, oxymorphone and their salts etc., and the like can be mentioned.

As the chemotherapeutic agents, for example, alkylating agents (e.g., cyclophosphamide, ifosphamide etc.), metabolic antagonists (e.g., methotrexate, 5-fluorouracil etc.), anticancer antibiotics (e.g., mitomycin, adriamycin etc.), plant-derived anticancer agents (e.g., vincristine, vindesine, taxol etc.), cisplatin, carboplatin, etoposide and the like can be mentioned. Of these, furtulon, neofurtulon etc., which are 5-fluorouracil derivatives, and the like are preferable.

As the immunotherapeutic agents, for example, microorganism or bacterial components (e.g., muramyl dipeptide derivative, picibanil etc.), polysaccharides having immunostimulant activity (e.g., lenthinan, schizophyllan, krestin etc.), cytokines obtained by genetic engineering techniques (e.g., interferon, interleukin (IL) etc.), colony stimulating factor (e.g., granulocyte-colony stimulating factor, erythropoietin etc.) and the like can be mentioned, with preference given to IL-1, IL-2, IL-12 and the like.

As the therapeutic agents for osteoporosis, for example, alfacalcidol, calcitriol, elcaltonin, calcitonin salmon, estriol, ipriflavone, pamidronate disodium, alendronate sodium hydrate, incadronate disodium and the like can be mentioned.

As the anti-dementia agents, for example, tacrine, donepezil, rivastigmine, galanthamine and the like can be mentioned.

As the erectile dysfunction amelioration agents, for example, apomorphine, sildenafil citrate and the like can be mentioned.

As the therapeutic agent for urinary incontinence/urinary frequency, for example, flavoxate hydrochloride, oxybutynin hydrochloride, propiverine hydrochloride and the like can be mentioned.

Moreover, pharmaceutical agents having a cachexia improving effect acknowledged in animal models and clinical situations, which include cyclooxygenase inhibitors (e.g., indomethacin etc.)[Cancer Research, Vol. 49, pp. 5935-5939, 1989], progesterone derivatives (e.g., megestrol acetate) [Journal of Clinical Oncology, Vol. 12, pp. 213-225, 1994], glucosteroid (e.g., dexamethasone etc.), metoclopramide pharmaceutical agents, tetrahydrocannabinol pharmaceutical agent (publications are the same as the above), fat metabolism improving agent (e.g., eicosapentanoic acid etc.)[British Journal of Cancer, Vol. 68, pp. 314-318, 1993], growth hormone, IGF-1, and antibodies against TNF-α, LIF, IL-6 and oncostatin M, which induce cachexia, and the like, can be also used in combination with the pharmaceutical agent of the present invention.

The combination drug preferably includes a diuretic, an insulin preparation, an insulin sensitizer, an α-glucosidase inhibitor, a biguanide agent, an insulin secretagogue (preferably sulfonylurea agent) and the like. Particularly, a diuretic such as hydrochlorothiazide and the like and an insulin sensitizers such as pioglitazone hydrochloride and the like are preferable.

The above-mentioned combination drug may be a combination of two or more kinds thereof combined at appropriate ratios.

Since compound A enhances hypoglycemic activity of other insulin sensitizers, a combined use of compound A, a salt thereof, or a prodrug thereof (particularly the compound of the present invention) and other insulin sensitizers (preferably pioglitazone hydrochloride) markedly enhances a prophylactic and/or therapeutic effect against diseases in which insulin resistance is involved, such as type II diabetes and the like.

### Examples

The present invention is explained in detail by referring to the following Examples, Preparation Examples and Experimental Examples. However, these Examples are mere practical embodiments and do not limit the present invention. The present invention may be modified as long as the scope of the invention is not deviated.

The elution by column chromatography in Examples was performed under observation by TLC (thin-layer chromatography). In the TLC observation, 60F₂₅₄ (manufactured by Merck) was used as a TLC plate, the solvent used as an elution solvent in the column chromatography was used as a developing solvent, and UV detector was used for detection. As silica gel for column chromatography, Kieselgel 60 (70-230 mesh) or Kieselgel 60 (230-400 mesh) manufactured by Merck was used. As basic silica gel, Chromatorex (NH) (100-200 mesh) manufactured by FUJI SILSIA CHEMICAL LTD. was used. NMR spectrum was measured by Bruker AVANCE 300 (300 MHz) using tetramethylsilane as an internal or external standard, and the chemical shift is expressed in δ value and the coupling constant is expressed in Hz. Powder X-ray crystal diffraction was measured using RINT2100 Ultima+/PC [cuκα rays (λ=1.5418 Å)] manufactured by Rigaku Corporation. The symbols in the Examples mean the following.

| | |
|---|---|
| s | : singlet |
| d | : doublet |
| t | : triplet |
| q | : quartet |
| dd | : double doublet |
| m | : multiplet |
| J | : coupling constant |
| THF | : tetrahydrofuran |
| DMF | : N,N-dimethylformamide |
| DMSO | : dimethyl sulfoxide |
| DBU | : 1,8-diazabicyclo[5.4.0]-7-undecene |
| DMAP | : 4-dimethylaminopyridine |
| JP1 | : Japan Pharmacopoeia (Fourteenth edition) disintegration test solution 1 |
| JP2 | : Japan Pharmacopoeia (Fourteenth edition) disintegration test solution 2 |
| GCDC/JP2: | Japan Pharmacopoeia disintegration test solution 2 containing glycochenodeoxycholic acid |

### Reference Example 1

### methyl 1-[(2'-cyanobiphenyl-4-yl)methyl]-2-cyclopropyl-1H-benzimidazole-7-carboxylate

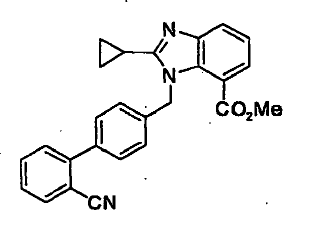

Methyl 3-amino-2-{[(2'-cyanobiphenyl-4-yl)methyl]amino}benzoate (42 g) was dissolved in ethyl acetate (420 ml), and triethylamine (19.7 ml) was added. Cyclopropanecarbonyl chloride (12.2 ml) was added dropwise at 0°C, and the mixture was stirred for 6 hrs. Water was added and the mixture was extracted with ethyl acetate. The organic layer was washed successively with saturated aqueous sodium hydrogencarbonate and saturated brine, dried over magnesium sulfate, and concentrated. The residue was dissolved in ethanol (380 ml), then concentrated hydrochloric acid (42 ml) was added, and the mixture was stirred at 80°C for 5 hrs. Aqueous sodium hydroxide solution was added to neutralize the mixture, and the mixture was extracted with ethyl acetate. The organic layer was washed successively with saturated aqueous sodium hydrogencarbonate and saturated brine, dried over magnesium sulfate, and concentrated. The obtained crystals were washed with diisopropyl ether to give the title compound (46.2 g, 96%).
1H NMR (300 MHz, CDCl₃) δ ppm 1.05 - 1.14 (m, 2 H) , 1.22 - 1.30 (m, 2 H), 1.93 - 2.05 (m, 1 H) , 3.73 (s, 3 H), 5.97 (s, 2 H), 7.06 (d, J=8.48 Hz, 2 H), 7.19 - 7.29 (m, 1 H), 7.38 - 7.50 (m, 4 H) , 7.57 - 7.69 (m, 2 H) , 7.72 - 7.78 (m, 1 H) , 7.89 (dd, J=7.91, 1.13 Hz, 1 H).

### Reference Example 2

### methyl 2-cyclopropyl-1-{[2'-(5-oxo-4,5-dihydro-1,2,4-oxadiazol-3-yl)biphenyl-4-yl]methyl}-1H-benzimidazole-7-carboxylate

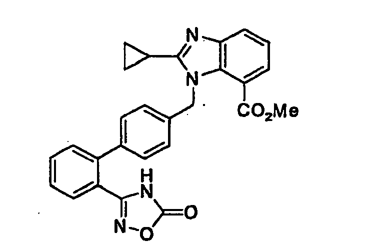

Hydroxylamine hydrochloride (78.8 g) was dissolved in DMSO (500 ml), and sodium hydrogencarbonate (114 g) was added, and the mixture was stirred at 50°C for 50 min. The compound (46.2 g) obtained in Reference Example 1 was added, and the mixture was stirred at 80°C for 12 hrs. Water was added to the reaction mixture, and the mixture was extracted with ethyl acetate. The organic layer was washed with saturated brine, dried over magnesium sulfate, and concentrated. The residue was dissolved in THF (436 ml), and carbonyldiimidazole (19.3 g) and DBU (11.9 ml) were added, and the mixture was stirred for 30 min. Water was added and the mixture was extracted with ethyl acetate. The organic layer was washed successively with saturated aqueous sodium hydrogencarbonate and saturated brine, dried over magnesium sulfate, and concentrated. The residue was purified by basic silica gel chromatography to give the title compound (44.0 g, 83%).
1H NMR (300 MHz, DMSO-d₆) δ ppm 0.99 - 1.12 (m, 4 H), 2.20 - 2.32 (m, 1 H), 3.67 (s, 3 H), 5. 86 (s, 2 H), 6.96 (d, J=8.10 Hz, 2 H), 7.18 - 7.29 (m, 3 H), 7.44 - 7.59 (m, 3 H), 7.62 - 7.71 (m, 2 H), 7.79 (d, J=7.91 Hz, 1 H), 12.39 (s, 1 H).

### Reference Example 3

### 2-cyclopropyl-1-{[2'-(5-oxo-4,5-dihydro-1,2,4-oxadiazol-3-yl)biphenyl-4-yl]methyl}-1H-benzimidazole-7-carboxylic acid

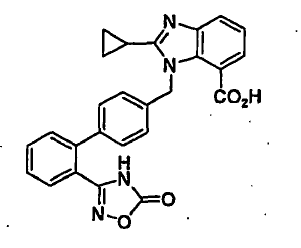

The compound (31.8 g) obtained in Reference Example 2 was dissolved in 0.4N aqueous sodium hydroxide solution (673 ml), and the mixture was stirred at 70°C for 5 hrs. 1N Hydrochloric acid (270 ml) was added dropwise, and the precipitated crystals were collected by filtration to give the title compound (30.8 g, 97%).
1H NMR (300 MHz, DMSO-d₆) δ ppm 0.95 - 1.08 (m, 4 H) , 2.17 - 2.30 (m, 1 H), 6.03 (s, 2 H), 6.99 (d, J=8.29 Hz, 2 H), 7.19 - 7.26 (m, 3 H), 7.43 - 7.70 (m, 5 H), 7.76 (dd, J=7.91, 1.13 Hz, 1 H).

### Example 1

### (5-methyl-2-oxo-1,3-dioxol-4-yl)methyl 2-cyclopropyl-1-{[2'-(5-oxo-4,5-dihydro-1,2,4-oxadiazol-3-yl)biphenyl-4-yl]methyl}-1H-benzimidazole-7-carboxylate

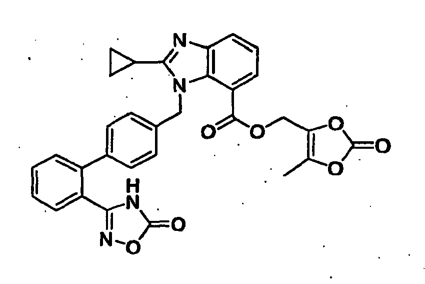

The compound (4.20 g) obtained in Reference Example 3 was dissolved in THF (42 ml), and triethylamine (1.42 ml) and 2,4,6-trichlorobenzoyl chloride (1.52 ml) were added, and the mixture was stirred for 12 hrs. The insoluble materials were removed by filtration, and the filtrate was concentrated. The residue was dissolved in dichloromethane (42 ml), and medoxomil alcohol (1.45 g) and DMAP (1.36 g) were added, and the mixture was stirred for 12 hrs. The reaction mixture was diluted with chloroform, washed successively with 1N hydrochloric acid, saturated aqueous sodium hydrogencarbonate and saturated brine. The organic layer was dried over magnesium sulfate and concentrated to give the title compound (3.08 g, 59%).
1H NMR (300 MHz, DMSO-d₆) δ ppm 0.97 - 1.10 (m, 4 H) , 2.14 (s, 3 H), 2.18 - 2.31 (m, 1 H), 5.11 (s, 2 H), 5.89 (s, 2 H) , 6.96 (d, J=8.29 Hz, 2 H), 7.18 - 7.31 (m, 3 H), 7.44 - 7.71 (m, 5 H), 7.82 (dd, J=8.01, 1.04 Hz, 1 H), 12.38 (s, 1 H).

### Example 2

### potassium 3-{4'-[(2-cyclopropyl-7-{[(5-methyl-2-oxo-1,3-dioxol-4-yl)methoxy]carbonyl}-1H-benzimidazol-1-yl)methyl]biphenyl-2-yl}-1,2,4-oxadiazol-5-ate

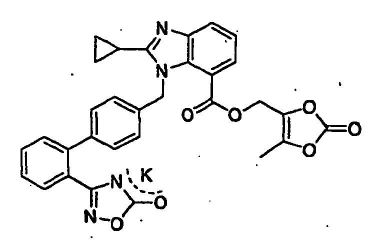

The compound (1.00 g) obtained in Example 1 was dissolved in acetone (20 ml), and potassium 2-ethylhexanoate (0.323 g) was added, and the mixture was stirred for 4 hrs and 30 min. The precipitated crystals were collected by filtration to give the title compound (0.581 g, 54%)
1H NMR (300 MHz, DMSO-d₆) δ ppm 1.08 (d, 4 H, J=6.2 Hz), 2.15 (s, 3 H), 2.25 - 2.34 (m, 1 H), 5.09 (s, 2 H), 5.84 (s, 2 H), 6.82 (d, 2 H, J=8.3 Hz), 7.18 - 7.28 (m, 4 H), 7.29 - 7.42 (m, 2 H), 7.45 - 7.50 (m, 1 H), 7.53 (dd, 1 H, J=7.5, 1.1 Hz), 7.80 (dd, 1 H, J=7.9, 1.1 Hz).

### Example 3

### crystal of (5-methyl-2-oxo-1,3-dioxol-4-yl)methyl 2-cyclopropyl-1-{[2'-(5-oxo-4,5-dihydro-1,2,4-oxadiazol-3-yl)biphenyl-4-yl]methyl}-1H-benzimidazole-7-carboxylate

The compound obtained in Example 1 was recrystallized from acetonitrile to give a solvate crystal containing acetonitrile. This was dried overnight at 100°C under reduced pressure to give Form A crystal, which is stable to heat and practical. The obtained crystal had a powder X-ray crystal diffraction pattern shown in Fig. 1, and approximately the following diffraction angles.

**Table 1**

| Diffraction angle (2θ) | Relative intensity |
|---|---|
| 5.08 | 34 |
| 10.10 | 62 |
| 11.52 | 38 |
| 11.62 | 38 |
| 14.76 | 50 |
| 15.56 | 41 |
| 15.68 | 59 |
| 17.10 | 100 |
| 17.20 | 76 |
| 19.74 | 46 |
| 21.00 | 59 |
| 21.18 | 60 |
| 21.30 | 63 |
| 23.50 | 51 |
| 23.82 | 41 |
| 23.94 | 50 |
| 24.12 | 63 |
| 24.20 | 44 |
| 25.02 | 43 |
| 25.44 | 60 |
| 25.76 | 42 |
| 25.86 | 51 |

### Example 4

### crystal of (5-methyl-2-oxo-1,3-dioxol-4-yl)methyl 2-cyclopropyl-1-{[2'-(5-oxo-4,5-dihydro-1,2,4-oxadiazol-3-yl)biphenyl-4-yl]methyl}-1H-benzimidazole-7-carboxylate

2-Cyclopropyl-1-{[2'-(5-oxo-4,5-dihydro-1,2,4-oxadiazol-3-yl)biphenyl-4-yl]methyl}-1H-benzimidazole-7-carboxylic acid (1 kg) was dissolved in N,N-dimethylacetamide (10 L), and 4-hydroxymethyl-5-methyl-1,3-dioxol-2-one (345 g) was added. After cooling to not more than 10°C, p-toluenesulfonyl chloride (463 g), 4-dimethylaminopyridine (54 g) and potassium carbonate (397 g) were added, and the mixture was stirred at not more than 20°C for about 3 hrs. 0.5N Hydrochloric acid was added to adjust pH to 4, and water (10 L) was added to allow crystallization. The precipitated crystals were collected by filtration under reduced pressure, and washed successively with N,N-dimethylacetamide (2 L) and 70% water-containing acetone (2 L). The isolated crystals were suspended in 14% water-containing acetone (6 L), and the suspension was dissolved by heating to 50°C. Activated carbon (30 g) was added, and the mixture was stirred for 10 min. The activated carbon was removed by filtration, and washed with 14% water-containing acetone (1 L). The filtrate was cooled to about 25°C to allow precipitation of crystals, and the mixture was stirred at the same temperature for 1 hr. Water (13 L) was added and the mixture was further stirred for 1 hr. The mixture was cooled to not more than 10°C and further stirred for 1 hr. The precipitated crystals were isolated and washed with 70% water-containing acetone (6 L) to give a solvate crystal containing acetone. The crystal was dried at 90°C under reduced pressure to give Form A crystal (903 g, yield: 80%).

### Example 5

### crystal of (5-methyl-2-oxo-1,3-dioxol-4-yl)methyl 2-cyclopropyl-1-{[2'-(5-oxo-4,5-dihydro-1,2,4-oxadiazol-3-yl)biphenyl-4-yl]methyl}-1H-benzimidazole-7-carboxylate

2-Cyclopropyl-1-{[2'-(5-oxo-4,5-dihydro-1,2,4-oxadiazol-3-yl)biphenyl-4-yl]methyl}-1H-benzoimidazole-7-carboxylic acid (15.00 kg) was dissolved in N,N-dimethylacetamide (150 L), and 4-hydroxymethyl-5-methyl-1,3-dioxol-2-one (5.18 kg) was added. After cooling to not more than 10°C, p-toluenesulfonyl chloride (6.95 kg), 4-dimethylaminopyridine (0.81 kg) and potassium carbonate (5.96 kg) were added, and the mixture was stirred at not more than 20°C for about 3 hrs. 0.5N Hydrochloric acid was added to adjust pH to 4, and water (150 L) was added to allow crystallization. The crystallized solvate crystals were collected by filtration under reduced pressure, and washed successively with N,N-dimethyladetamide (30 L) and 70% water-containing acetone (30 L). The isolated crystals were suspended in 40% water-containing acetone (225 L), and the suspension was dissolved by heating to 50°C. The solution was decontaminated by filtration, and washed with 50% water-containing acetone (30 L). The filtrate was cooled to about 25°C to allow precipitation of crystals, and the mixture was stirred at the same temperature for 1 hr. Water (45 L) was added and the mixture was further stirred for 1 hr. The mixture was cooled to not more than 10°C and further stirred for 1 hr. The precipitated crystals were isolated and washed with 50% water-containing acetone (30 L) to give a solvate crystal containing acetone. The crystal was dried at 95°C under reduced pressure to give Form A crystal (15.73 kg, yield: 84%).

### Formulation Examples

When the compound of the present invention is to be used as a therapeutic agent for circulatory diseases such as hypertension, cardiac disease, stroke, nephritis and the like, for example, the following formulation can be used.

In the following formulation, as the components (additive) other than the active ingredient, those listed in the Japanese Pharmacopoeia, the Japanese Pharmacopoeia quasi drugs or the pharmaceutical product additive standard, and the like can be used.

### 1. Tablet

| | |
|---|---|
| (1) Compound obtained in Example 4 | 10 mg |
| (2) Lactose | 35 mg |
| (3) Corn starch | 150 mg |
| (4) Microcrystalline cellulose | 30 mg |
| (5) Magnesium stearate | 5 mg |
| 1 tablet | 230 mg |

| | |
|---|---|
| (1), (2), (3), 2/3 of (4) and 1/2 of (5) are admixed and granulated. Thereto are added the remaining (4) and (5), and the mixture is compressed to give tablets. | |

### 2. Capsule

| | |
|---|---|
| (1) Compound obtained in Example 2 | 10 mg |
| (2) Lactose | 69.5 mg. |
| (3) Light silicic anhydride | 0.2 mg |
| (4) Magnesium stearate | 0.3 mg |
| 1 capsule | 80 mg |

| | |
|---|---|
| (1), (2), (3) and (4) were dry mixed and filled in HPMC capsule (No. 1). | |

### 3. Tablet

Compound (I) (17.24 g), mannitol (3342 g) and microcrystalline cellulose (663 g) were uniformly mixed in a fluidized bed granulating dryer, and the mixture was granulated in the dryer by spraying an aqueous solution of hydroxypropylcellulose (132.6 g) and dried therein. The obtained granules were pulverized with a 1.5 mmφ punching screen using a power mill to give a sized powder. To the sized powder (3788 g) were added croscarmellose sodium (Ac-Di-Sol) (201.5 g) and magnesium stearate (40.3 g) and they were mixed to give granules for tableting. The granules were tableted by a tableting machine with a 7.0 mmφ punch to give plain tablets weighing 130 mg per tablet. A hydroxypropylmethylcellulose 2910 solution obtained by dispersing titanium oxide and yellow ferric oxide and dissolving polyethylene glycol 8000 was sprayed on the obtained plain tablets in a film coating machine to give about 25000 film-coated tablets having the theoretical formulation shown in Table 2, which contain 0.5 mg of compound (I) per tablet.

**Table 2**

| Composition | Amount added (mg) |
|---|---|
| Compound (I) | 0.5 |
| Mannitol | 98.3 |
| Microcrystalline cellulose | 19.5 |
| Hydroxypropylcellulose | 3.9 |
| Croscarmellose sodium | 6.5 |
| Magnesium stearate | 1.3 |
| Plain tablet | 130 |
| Hydroxypropylmethylcellulose 2910 | 3.735 |
| Polyethylene glycol 8000 | 0.75 |
| Titanium oxide | 0.5 |
| Yellow ferric oxide | 0.015 |
| Total | 135 |

### 4. Tablet

Compound (I) (172.4 g), mannitol (3187 g) and microcrystalline cellulose (663 g) were uniformly mixed in a fluidized bed granulating dryer, and the mixture was granulated in the dryer by spraying an aqueous solution of hydroxypropylcellulose (132.6 g) and dried therein. The obtained granules were pulverized with a 1.5 mmφ punching screen using a power mill to give a sized powder. To the sized powder (3788 g) were added croscarmellose sodium (Ac-Di-Sol) (201.5 g) and magnesium stearate (40.3 g) and they were mixed to give granules for tableting. The granules were tableted by a tableting machine with a 7.0 mmφ punch to give plain tablets weighing 130 mg per tablet. A hydroxypropylmethylcellulose 2910 solution obtained by dispersing titanium oxide and yellow ferric oxide and dissolving polyethylene glycol 8000 was sprayed on the obtained plain tablets in a film coating machine to give about 25000 film-coated tablets having the theoretical formulation shown in Table 3, which contain 5 mg of compound (I) per tablet.

**Table 3**

| Composition | Amount added (mg) |
|---|---|
| Compound (I) | 5 |
| Mannitol | 93.8 |
| Microcrystalline cellulose | 19.5 |
| Hydroxypropylcellulose | 3.9 |
| Croscarmellose sodium | 6.5 |
| Magnesium stearate | 1.3 |
| Plain tablet | 130 |
| Hydroxypropylmethylcellulose 2910 | 3.735 |
| Polyethylene glycol 8000 | 0.75 |
| Titanium oxide | 0.5 |
| Yellow ferric oxide | 0.015 |
| Total | 135 |

### 5: Tablet

Compound (I) (689.7 g), mannitol (2670 g) and microcrystalline cellulose (663 g) were uniformly mixed in a fluidized bed granulating dryer, and the mixture was granulated in the dryer by spraying an aqueous solution of hydroxypropylcellulose (132.6 g) and dried therein. The obtained granules were pulverized with a 1.5 mmφ punching screen using a power mill to give a sized powder. To the sized powder (3788 g) were added croscarmellose sodium (Ac-Di-Sol) (201.5 g) and magnesium stearate (40.3 g) and they were mixed to give granules for tableting. The granules were tableted by a tableting machine with a 7.0 mmφ punch to give plain tablets weighing 130 mg per tablet. A hydroxypropylmethylcellulose 2910 solution obtained by dispersing titanium oxide and yellow ferric oxide and dissolving polyethylene glycol 8000 was sprayed on the obtained plain tablets in a film coating machine to give about 25000 film-coated tablets having the theoretical formulation shown in Table 4, which contain 20 mg of compound (I) per tablet.

**Table 4**

| Composition | Amount added (mg) |
|---|---|
| Compound (I) | 20 |
| Mannitol | 78.8 |
| Microrystalline cellulose | 19.5 |
| Hydroxypropylcellulose | 3.9 |
| Croscarmellose Sodium | 6.5 |
| Magnesium stearate | 1.3 |
| Plain tablet | 130 |
| Hydroxypropylmethylcellulose 2910 | 3.735 |
| Polyethylene glycol 8000 | 0.75 |
| Titanium oxide | 0.5 |
| Yellow ferric oxide | 0.015 |
| Total | 135 |

### 6. Tablet

Compound (I) (3.4 g), lactose (311.4 g) and corn starch (88.4 g) were uniformly mixed in a fluidized bed granulating dryer, and the mixture was granulated in the dryer by spraying an aqueous solution of hydroxypropylcellulose (13.26 g) and dried therein. The obtained granules were pulverized with a 1.5 mmφ punching screen using a power mill to give a sized powder. To the sized powder (306.3 g) were added croscarmellose sodium (Ac-Di-Sol) (16.25 g) and magnesium stearate (2.5 g) and they were mixed to give granules for tableting. The granules were tableted by a tableting machine with a 7.0 mmφ punch to give plain tablets weighing 130 mg per tablet. A hydroxypropylmethylcellulose 2910 solution obtained by dispersing titanium oxide and yellow ferric oxide and dissolving polyethylene glycol 8000 was sprayed on the obtained plain tablets in a film coating machine to give about 900 film-coated tablets having the theoretical formulation shown in Table 5, which contain 1 mg of compound (I) per tablet.

**Table 5**

| Composition | Amount added (mg) |
|---|---|
| Compound (I) | 1 |
| Lactose | 91.6 |
| Corn starch | 26 |
| Hydroxypropylcellulose | 3.9 |
| Croscarmellose sodium | 6.5 |
| Magnesium stearate | 1 |
| Plain tablet | 130 |
| Hydroxypropylmethylcellulose 2910 | 3.735 |
| Polyethylene glycol 8000 | 0.75 |
| Titanium oxide | 0.5 |
| Yellow ferric oxide | 0.015 |
| Total | 135 |

### Experimental Example 1

### Inhibitory effect of the compound of the present invention on angiotensin II (AII) -induced vasopressor action in rat

11-Week-old male SD rats (JCL: SD, CLEA Japan, Inc.) were anesthetized with pentobarbital (50 mg/kg, i.p.), the femoral artery and femoral vein were isolated; and a polyethylene tube filled with physiological saline containing heparin (200 U/mL) was placed therein. The catheter was subcutaneously fixed in the back of the neck. After recovery period, the rats were subjected to the test.

The arterial catheter was connected to a pressure transducer (2238, NEC San-ei Instruments) and the signals were output on a pen recorder (RECTI-HORIZ 8K, NEC San-ei Instruments) via an amplifier for blood pressure. After the vasopressor action of AII (100 ng/kg, i.v.) was stabilized, a test compound in an equimolar dose to compound A was administered. After 24 hrs, AII was administered, an increase in the blood pressure was measured, and an inhibition rate relative to the value before drug administration was calculated. All compounds were suspended in 0.5% methylcellulose and orally administered at a volume of 2 mL/kg,

The results are shown as mean±SEM (Table 6). The significance between the compound (I) administration group and other compound administration group was determined by the Student's t test (**: p<0.01, *: p<0.05).

**Table 6**

| | suppression rate at 24 hrs after administration |
|---|---|
| Compound (I) [0.12 mg/kg, p.o. (n=5)] | 26.2±3.2 % |
| Compound A [0.10 mg/kg, p.o. (n=5)] | -6.0±3.5 % ** |
| Compound B [0.10 mg/kg, p.o. (n=5)] | 2.6±4.8 % |

As is evident from the results, the compound of the present invention shows a sustained and strong pharmacological action by oral administration.

### Experimental Example 2

### Enhancing effect of the compound of the present invention on insulin sensitivity in rat

24-Week-old male spontaneously hypertensive rats (SLC: SHR/Izm, Japan SLC, Inc.) were used. The body weight, systolic blood pressure, fasting blood glucose level, plasma insulin level and plasma triglyceride level of the rats were measured, and the rats were divided into a vehicle (0.5% methylcellulose solution) administration group and compound (I) (0.12, 0.37 and 1.23 mg/kg) administration groups using them as indices. Compound (I) was suspended in 0.5% methylcellulose solution and orally administered at a volume of 2 mL/kg for 2 weeks.

The insulin sensitivity was evaluated by the glucose clamp technique. To be specific, the rats after fasting overnight were anesthetized with pentobarbital sodium (Nembutal injection, Dainippon Pharmaceutical Co., Ltd., 50 mg/kg i.p.), and catheters (SP45, Natume Seisakusho Co., Ltd.) for blood sampling, insulin (novolin R/100, Novo Nordisk Pharma Co., Ltd.) infusion and glucose (Otsuka Glucose Injection 50%, Otsuka Pharmaceutical Co., Ltd.) infusion were each placed in the right common carotid artery, left femoral vein and right femoral vein. After single intravenous injection of 25 mU/kg of insulin, high insulin state was maintained by infusion using an infusion pump (KDS100, KDS) at an injection rate of 4 mU/kg/min. In addition, glucose in an amount necessary for maintaining the normal blood glucose level was intravenously infused using a different infusion pump (KDS100, KDS). Intravenous injection of glucose was started from 10 min after the start of the injection of insulin, and the glucose injection rate was changed after blood glucose measurement performed every 5 min. The blood glucose level then was quickly measured using a simple blood glucose measurement apparatus (ACCU-CHEK Comfort, Roche diagnostics). Glucose was infused for 90 min., the average value of the glucose injection rate was calculated for 40 min (from 50 min. to 90 min. after the start of the injection) and used as an index (M value) of insulin sensitivity.

The results are shown as mean±SEM (Table 7). For comparison of the vehicle administration group and the compound (I) administration group, Williams test was used (*: p<0.025).

**Table 7**

| | M value |
|---|---|
| Vehicle administration group (n=11) | 6.7±0.7 |
| Compound (I) [0.12 mg/kg, p.o.(n=12)] | 8.2±0.8 |
| Compound (I) [0.37 mg/kg, p.o.(n=12)] | 9.0±0.6 * |
| Compound (I) [1.23 mg/kg, p.o.(n=11)] | 9.8±0.8 * |

As is evident from the results, the compound of the present invention shows a strong enhancing action of insulin sensitivity by oral administration.

### Experimental Example 3

### Evaluation of solubility and membrane permeability by artificial membrane permeation test (Parallel Artificial Membrane Permeability Assay; PAMPA)

### (1) Solubility

About 2 mg of samples were suspended in 2 mL of JP1, JP2 and 20 mmol/L GCDC/JP2. The suspension was equilibrated at 37°C for 2 hrs and filtered. The concentration in the solutions was determined by HPLC under the following conditions.

### HPLC conditions

| | | |
|---|---|---|
| Detector: | UV 254 nm | |
| Column: | CAPCELLPAK C18 MG 75x4.6mm | |
| Mobile Phase A: | 0.05 mol/L ammonium formate buffer (pH 3) | |
| | /MeCN = 9:1 | |
| Mobile Phase B: | 0.05 mol/L ammonium formate buffer (pH 3) | |
| | /MeCN = 1:9 | |
| Gradient Program: | 0→10 min | 0→100% B) |
| | 10→15 min | 100% B) |
| | 15.1→20 min | 0% B) |
| Column Temp.: | 40°C | |
| Flow Rate: | 1 mL/min | |
| Injection vol.: | 10 µL | |

### (2) Membrane permeability

The permeability with the artificial membrane was determined by PAMPA under the following conditions.

| | |
|---|---|
| lipid membrane: | GIT mode (pION) |
| measurement wavelength: | 250-400 nm |
| incubation time: | 3 hrs |
| incubation temperature: | 25°C |
| Donor: | buffer containing 10% DMSO |
| pH: | 3 points of 7.4, 6.0, 5.5 |
| compound concentration: | 50 µmol/L |

The results of solubility and permeability of compound X (Form A crystal of compound (I)), compound A and compound B are shown in Table 8.

**Table 8**

| T-No. | Solubility (µg/mL) | | | PAMPA (nm/s) | | |
|---|---|---|---|---|---|---|
| | JP1 | JP2 | GCDC/JP2 | PH 7.4 | PH 6.2 | pH 5.5. |
| Compound X | 27 | 8.3 | 52 | 95 | 190 | 210 |
| Compound A | 130 | 470 | 680 | 0 | 6.3 | 130 |
| Compound B | 160 | 2.8 | 27 | 89 | 200 | 460 |

In comparison with compound X, compound A was more soluble but less permeable and compound B was more permeable but less soluble. Compound A is considered to cause poor oral absorption due to the low permeability. Compound B is also considered to cause poor oral absorption due to the rate limiting low solubility.

Therefore, compound X is expected to achieve higher oral absorption as compared to compound A and compound B.

### Industrial Applicability

The compound of the present invention is useful as a drug for the prophylaxis or treatment of circulatory diseases such as hypertension and the like and metabolic diseases such as diabetes and the like, and the like.

## Claims

1. (5-Methyl-2-oxo-1,3-dioxol-4-yl)methyl 2-cyclopropyl-1-{[2'-(5-oxo-4,5-dihydro-1,2,4-oxadiazol-3-yl)biphenyl-4-yl]methyl}-1H-benzimidazole-7-carboxylate.

2. A salt of (5-methyl-2-oxo-1,3-dioxol-4-yl)methyl 2-cyclopropyl-1-{[2'-(5-oxo-4,5-dihydro-1,2,4-oxadiazol-3-yl)biphenyl-4-yl]methyl}-1H-benzimidazole-7-carboxylate.

3. (5-Methyl-2-oxo-1,3-dioxol-4-yl)methyl 2-cyclopropyl-1-{[2'-(5-oxo-4,5-dihydro-1,2,4-oxadizol-3-yl)biphenyl-4-yl]methyl}-1H-benzimidazole-7-carboxylate potassium salt.

4. A solvate of (5-methyl-2-oxo-1,3-dioxol-4-yl)methyl 2-cyclopropyl-1-{[2'-(5-oxo-4,5-dihydro-1,2,4-oxadiazol-3-yl)biphenyl-4-yl]methyl}-1H-benzimidazole-7-carboxylate.

5. A crystal of the compound of any of claims 1 to 4.

6. A method for producing (5-methyl-2-oxo-1,3-dioxol-4-yl)methyl 2-cyclopropyl-1-{[2'-(5-oxo-4,5-dihydro-1,2,4-oxadiazol-3-yl)biphenyl-4-yl]methyl}-1H-benzimidazole-7-carboxylate or a salt thereof, which comprises reacting a reactive derivative of 2-cyclopropyl-1-{[2'-(5-oxo-4,5-dihydro-1,2,4-oxadiazol-3-yl)biphenyl-4-yl]methyl}-1H-benzimidazole-7-carboxylic acid, or a salt thereof, with 4-hydroxymethyl-5-methyl-1,3-dioxol-2-one or a salt thereof.

7. A pharmaceutical agent comprising the compound of any of claims 1 to 4.

8. The pharmaceutical agent of claim 7, which is an angiotensin II antagonist.

9. The pharmaceutical agent of claim 7, which is an agent for the prophylaxes or treatment of circulatory diseases.

10. An insulin sensitizer comprising the compound of any of claims 1 to 4.

11. An enhancer of a hypoglycemic activity of an insulin sensitizer, which comprises the compound of any of claims 1 to 4.

12. A compound according to any of claims 1 to 4 for its use to antagonize angiotensin II in a mammal.

13. Use of the compound of any of claims 1 to 4 for the production of an angiotensin 11 antagonist.

14. A compound according to any of claims 1 to 4 for its use to prevent or treat circulatory diseases in an animal.

15. Use of the compound of any of claims 1 to 4 for the production of an agent for the prophylaxis or treatment of circulatory diseases.

16. A compound of any of claims 1 to 4 for its use to produce an insuline sensitizer.

17. Use of the compound of any of claims 1 to 4 for the production of an insulin sensitizer.

18. A compound of any of claims 1 to 4 for its use to produce an enhancer of a hypoglycemic activity of an insulin sensitizer.

19. Use of the compound of any of claims 1 to 4 for the production of an enhancer of a hypoglycemic activity of an insulin sensitizer.

## Patentansprüche

1. (5-Methyl-2-oxo-1,3-dioxol-4-yl)methyl 2-cyclopropyl-1-{[2'-(5-oxo-4,5-dihydro-1,2,4-oxadiazol-3-yl)biphenyl-4-yl]methyl}-1H-benzimidazol-7-carboxylat.

2. Salz von (5-Methyl-2-oxo-1,3-dioxol-4-yl)methyl 2-cyclopropyl-1-{[2'-(5-oxo-4,5-dihydro-1,2,4-oxadiazol-3-yl)biphenyl-4-yl]methyl}-1H-benzimidazol-7-carboxylat.

3. (5-Methyl-2-oxo-1,3-dioxol-4-yl)methyl 2-cyclopropyl-1-{[2'-(5-oxo-4,5-dihydro-1,2,4-oxadiazol-3-yl)biphenyl-4-yl]methyl}-1H-benzimidazol-7-carboxylat-Kaliumsalz.

4. Solvat von (5-Methyl-2-oxo-1,3-dioxol-4-yl)methyl 2-cyclopropyl-1-{[2'-(5-oxo-4,5-dihydro-1,2,4-oxadiazol-3-yl)biphenyl-4-yl]methyl}-1H-benzimidazol-7-carboxylat.

5. Kristall der Verbindung nach einem der Ansprüche 1 bis 4.

6. Verfahren zur Herstellung von (5-Methyl-2-oxo-1,3-dioxol-4-yl)methyl 2-cyclopropyl-1-{[2'-(5-oxo-4,5-dihydro-1,2,4-oxadiazol-3-yl)biphenyl-4-yl]methyl}-1H-benzimidazol-7-carboxylat oder einem Salz davon, bei dem ein reaktionsfähiges Derivat von 2-Cyclopropyl-1-{[2'-(5-oxo-4,5-dihydro-1,2,4-oxadiazol-3-yl)biphenyl-4-yl)methyl}-1H-benzimidazol-7-carbonsäure oder ein Salz davon mit 4-Hydroxymethyl-5-methyl-1,3-dioxol-2-on oder einem Salz davon umgesetzt wird.

7. Pharmazeutisches Mittel, das die Verbindung nach einem der Ansprüche 1 bis 4 umfasst.

8. Pharmazeutisches Mittel nach Anspruch 7, das ein Angiotensin-II-Antagonist ist.

9. Pharmazeutisches Mittel nach Anspruch 7, das ein Mittel zur Prophylaxe oder Behandlung von Kreislauferkrankungen ist.

10. Insulinsensibilisator, der die Verbindung nach einem der Ansprüche 1 bis 4 umfasst.

11. Verstärker einer hypoglykämischen Wirkung eines Insulinsensibilisators, der die Verbindung nach einem der Ansprüche 1 bis 4 umfasst.

12. Verbindung nach einem der Ansprüche 1 bis 4 für deren Verwendung zum Antagonisieren von Angiotensin II bei einem Säugetier.

13. Verwendung der Verbindung nach einem der Ansprüche 1 bis 4 zur Herstellung eines Angiotensin-II-Antagonisten.

14. Verbindung nach einem der Ansprüche 1 bis 4 für deren Verwendung zur Vorbeugung oder Behandlung von Kreislauferkrankungen bei einem Tier.

15. Verwendung der Verbindung nach einem der Ansprüche 1 bis 4 für die Herstellung eines Mittels zur Prophylaxe oder Behandlung von Kreislauferkrankungen.

16. Verbindung nach einem der Ansprüche 1 bis 4 für deren Verwendung zur Herstellung eines Insulinsensibilisators.

17. Verwendung der Verbindung nach einem der Ansprüche 1 bis 4 zur Herstellung eines Insulinsensibilisators.

18. Verbindung nach einem der Ansprüche 1 bis 4 für deren Verwendung zur Herstellung eines Verstärkers einer hypoglykämischen Wirkung eines Insulinsensibilisators.

19. Verwendung der Verbindung nach einem der Ansprüche 1 bis 4 zur Herstellung eines Verstärkers einer hypoglykämischen Wirkung eines Insulinsensibilisators.

## Revendications

1. 2-Cyclopropyl-1-{[2'-(5-oxo-4,5-dihydro-1,2,4-oxadiazol-3-yl)-biphényl-4-yl]-méthyl}-1H-bendimidazole-7-carboxylate de (5-méthyl-2-oxo-1,3-dioxol-4-yl)-méthyle.

2. Sel de 2-cyclopropyl-1-{[2'-(5-oxo-4,5-dihydro-1,2,4-oxadiazol-3-yl)-biphényl-4-yl]-méthyl }-1H-bendimidazole-7-carboxylate de (5-méthyl-2-oxo-1,3-dioxol-4-yl)-méthyle.

3. Sel de potassium de 2-cyclopropyl-1-{[2'-(5-oxo-4,5-dihydro-1,2,4-oxadiazol-3-yl)-biphényl-4-yl]-méthyl}-1H-bendimidazole-7-carboxylate de (5-méthyl-2-oxo-1,3-dioxol-4-yl)-méthyle.

4. Solvat de 2-cyclopropyl-1-{[2'-(5-oxo-4,5-dihydro-1,2,4-oxadiazol-3-yl)-biphényl-4-yl]-méthyl}-1H-bendimidazole-7-carboxylate de (5-méthyl-2-oxo-1,3-dioxol-4-yl)-méthyle.

5. Cristal d'un composé conforme à l'une des revendications 1 à 4.

6. Procédé de préparation du 2-cyclopropyl-1-{[2'-(5-oxo-4,5-dihydro-1,2,4-oxadiazol-3-yl)-biphényl-4-yl]-méthyl}-1H-bendimidazole-7-carboxylate de (5-méthyl-2-oxo-1,3-dioxol-4-yl)-méthyle ou d'un sel de ce composé, qui comporte le fait de faire réagir un dérivé réactif de l'acide 2-cyclopropyl-1-{[2'-(5-oxo-4,5-dihydro-1,2,4-oxadiazol-3-yl)-biphényl-4-yl]-méthyl}-1H-bendimidazole-7-carboxylique, ou un sel d'un tel dérivé, avec de la 4-hydroxyméthyl-5-méthyl-1,3-dioxol-2-one ou avec un sel de ce composé.

7. Agent pharmaceutique comprenant un composé conforme à l'une des revendications 1 à 4.

8. Agent pharmaceutique conforme à la revendication 7, qui est un antagoniste de l'angiotensine II.

9. Agent pharmaceutique conforme à la revendication 7, qui est un agent conçu pour la prophylaxie ou le traitement de maladies circulatoires.

10. Agent de sensibilisation à l'insuline, comprenant un composé conforme à l'une des revendications 1 à 4.

11. Agent renforçant l'activité hypoglycémique d'un agent de sensibilisation à l'insuline, comprenant un composé conforme à l'une des revendications 1 à 4.

12. Composé conforme à l'une des revendications 1 à 4, pour son utilisation en vue de contrarier les effets de l'angiotensine II chez un mammifère.

13. Utilisation d'un composé conforme à l'une des revendications 1 à 4, en vue de la production d'un antagoniste de l'angiotensine II.

14. Composé conforme à l'une des revendications 1 à 4, pour son utilisation en vue de prévenir ou traiter des maladies circulatoires chez un animal.

15. Utilisation d'un composé conforme à l'une des revendications 1 à 4, en vue de la production d'un agent conçu pour la prophylaxie ou le traitement de maladies circulatoires.

16. Composé conforme à l'une des revendications 1 à 4, pour son utilisation en vue de produire un agent de sensibilisation à l'insuline.

17. Utilisation d'un composé conforme à l'une des revendications 1 à 4, en vue de la production d'un agent de sensibilisation à l'insuline.

18. Composé conforme à l'une des revendications 1 à 4, pour son utilisation en vue de produire un agent renforçant l'activité hypoglycémique d'un agent de sensibilisation à l'insuline.

19. Utilisation d'un composé conforme à l'une des revendications 1 à 4, en vue de la production d'un agent renforçant l'activité hypoglycémique d'un agent de sensibilisation à l'insuline.
